# EUROPEAN PATENT SPECIFICATION

(11) **EP 1 789 091 B1**
(45) Date of publication and mention of the grant of the patent: **25.08.2010**
(21) Application number: 05781464.2
(22) Date of filing: 26.08.2005
(51) Int. Cl.: A61K 47/48, A61P 37/08

(54) **PRODRUGS CONTAINING NOVEL BIO-CLEAVABLE LINKERS**
PRODRUGS MIT NEUEN BIOSPALTBAREN VERBINDERN
PROMEDICAMENTS CONTENANT DE NOUVEAUX LIENS BIO-CLIVABLES

(30) Priority: 26.08.2004 US 604632 P; 01.07.2005 IN MU07792005
(43) Date of publication of application: 30.05.2007
(60) Divisional application: 09157001.0
(73) Proprietor: Piramal Life Sciences Limited, Mumbai 400 013, Maharashtra (IN); Satyam, Apparao, Goregaon (East), Mumbai 400 063 (IN)
(72) Inventor: SATYAM, Apparao Nicholas Piramal Research Centre, Goregaon(East),Mumbai 400 063 (IN)
(74) Representative: Sanderson, Nigel Paul
(86) International application number: PCT/IB2005/052797
(87) International publication number: WO 2006/027711

(56) References cited:
- WO-A-01/13957
- WO-A-03/040104
- WO-A-2004/039771
- WO-A-2004/069159
- US-A1- 2003 044 845
- US-A1- 2005 002 942
- US-B1- 6 566 509
- IGNARRO L J ET AL: "NITRIC OXIDE DONORS AND CARDIOVASCULAR AGENTS MODULATING THE BIOACTIVITY OF NITRIC OXIDE" CIRCULATION RESEARCH, GRUNE AND STRATTON, BALTIMORE, US, vol. 90, no. 1, 11 January 2002 (2002-01-11), pages 21-28, XP008051659 ISSN: 0009-7330 cited in the application
- SHARMA M ET AL: "Bis[3-(4'-substituted phenyl)prop-2-ene]disulfides as a new class of antihyperlipidemic compounds" BIOORGANIC & MEDICINAL CHEMISTRY LETTERS, OXFORD, GB, vol. 14, no. 21, 1 November 2004 (2004-11-01), pages 5347-5350, XP004580528 ISSN: 0960-894X
- MAHGOUB H ET AL.: "Spectrophotometric determination of binary mixtures of pseudoephedrine with some H1-receptor antagonists using derivative ratio spectrum method" JOURNAL OF PHARMACEUTICAL AND BIOMEDICAL ANALYSIS, vol. 31, 2003, pages 801-809, XP002393773
- ASSELINE U. ET AL: 'Solide-Phase Synthesis of Modified Oligodeoxyribonucleotides with an Acridine Derivative or a Thiophosphate Group at their 3' end' TETRAHEDRON LETTERS vol. 30, no. 19, 1989, pages 2521 - 2524, XP002117188
- VAN RENSURG N.J.J. ET AL ARCHIVES OF BIOCHEMISTRY AND BIOPHYSICS vol. 118, no. 531, 1967, pages 531 - 535, XP024754075
- NEVALAINEN V.; POHJALA E. JOURNAL OF CHEMICAL RESEARCH SYNOPSES vol. 6, 1988, pages 182 - 183

## Description

### Field of the Invention

The present invention relates to compositions of prodrugs, including double prodrugs and mutual prodrugs, containing bio-labile linkers and linkages according to the claims processes for their preparation and pharmaceutical compositions containing them and their use.

### Background of the Invention

A prodrug is an active drug chemically transformed into a *per se* inactive derivative which by virtue of chemical or enzymatic attack is converted to the parent drug within the body before or after reaching the site of action. The process of converting an active drug into inactive form is called drug latentiation. Prodrugs can be carrier-linked-prodrugs and bioprecursors. The carrier-linked prodrug results from a temporary linkage of the active molecule with a transport moiety. Such prodrugs are less active or inactive compared to the parent active drug. The transport moiety will be chosen for its non-toxicity and its ability to ensure the release of the active principle with efficient kinetics. Whereas the bioprecursors result from a molecular modification of the active principle itself by generation of a new molecule that is capable of being a substrate to the metabolizing enzymes releasing the active principle as a metabolite.

Prodrugs are prepared to alter the drug pharmacokinetics, improve stability and solubility, decrease toxicity, increase specificity, and increase duration of the pharmacological effect of the drug. By altering pharmacokinetics the drug bioavailability is increased by increasing absorption, distribution, biotransformation, and excretion of the drug. Limited intestinal absorption, distribution, fast metabolism, and toxicity are some of the causes of failure of drug candidates during development. Avoidance of the foreseeable or proven pharmacokinetic defects thus assumes considerable significance in drug research. Accordingly, prodrugs play a significant role in drug research as well.

In designing the prodrugs, it is important to consider the following factors: a) the linkage between the carrier and the drug is usually a covalent bond, b) the prodrug is inactive or less active than the active principle, c) the prodrug synthesis should not be expensive, d) the prodrug has to be reversible or bioreversible derivative of the drug, and e) the carrier moiety must be non-toxic and inactive when released.

Prodrugs are usually prepared by: a) formation of ester, hemiesters, carbonate esters, nitrate esters, amides, hydroxamic acids, carbamates, imines, mannich bases, and enamines of the active drug, b) functionalizing the drug with azo, glycoside, peptide, and ether functional groups, c) use of polymers, salts, complexes, phosphoramides, acetals, hemiacetals, and ketal forms of the drug. For example, see Andrejus Korolkovas's, "Essentials of Medicinal Chemistry", pp. 97-118.

US5767134 and US20050002942A1 disclosed a few disulfide-contaming prodrugs/folate-drug conjugates. WO 9842661, US 5807847, WO 0054756 and WO 0149275 reported a few nitrooxy derivatives of organic molecules containing sulfahydryl or disulfide group which are called "SS-nitrates".

Representative examples from WO 9842661 have shown superior vasorelaxant activity and no tolerance was observed to the cGMP-increasing effects of those compounds under the same experimental conditions used for the induction of in vivo tolerance. WO 0149275 reports drug conjugates where an anti-inflammatory drug is covalently linked to the beta-mercapto-nitrate via thioester bond. Biotransformation pathways proposed for NO release from GTN have largely been heme-dependent or sulfahydryl-dependent. See, for examples, Thatcher, G. R. J. et al., Chem.Soc. Rev. 1998, 27, 331 and reference cited therein, and Bennett, B M. et al., Trends Pharmacol, Sci. 1994, 15, 245.

WO 03/040104 describes dimeric pharmaceutical compounds containing a linker. The dimeric compounds leads to increased potency, selectivity and long residence time particularly at epithelial surfaces in comparison to the respective monomer. The linker may be a hydrocarbon which includes N, O, S, amide, amine, carbonyl and/or carboxy functional groups.

WO 2004/039771 describes diallyldisulphide compounds having antilipidemic and antioxidant activity.

Biorganic & Medicinal Chemistry Letters, Vol. 14, No. 21, 2004, pages 5347 - 5350; discloses bis[3-(4'-substituted phenyl)-prop-2-ene]disulfides as a new class of hyperlipidemic compounds.

WO 2004/069159 describes a vitamin receptor binding drug delivery conjugate, which consists of a vitamin, or a vitamin receptor analog and a bivalent linker and a drug. Tetrahedron letters, Vol. 30, No. 19, pgs. 2521 - 2521 (1989) discloses use of a derivatised support involving the 2,2'-diethyldithio-group 7 to synthesise oligodeoxyribonucleotides bearing acridine derivative.

Archives of Biochemistry And Biophysics, Vol. 118, No. 531 (1967), pages 531- 535; discloses a suitable method of investigation of the reaction of sulfite on fifteen unsymmetrical alkyl-alkyl disulfides.

J. Of Chem. Research (s); 1988, 182-183; discloses a study of the reactions involved in the formation of dihydro-1,4-oxathiines by halogenation of 1,3-oxathiolanes.

A mutual prodrug is the association in a unique molecule of two drugs, usually synergistic, attached to each other, one drug being the carrier for the other and vice versa. The embodiments of the invention also provide mutual prodrugs, which are prodrugs of two therapeutic agents currently used/potential for use in combination therapy utilizing novel bio-cleavable linkers, water-soluble prodrugs of insoluble/sparingly-soluble therapeutic agents using the same linker technology and water-soluble double and prodrugs of sparingly-soluble therapeutic agents using the same linker technology.

According to a first aspect of the present invention there is provided a compound of formula (I), or pharmaceutically acceptable salts thereof,: 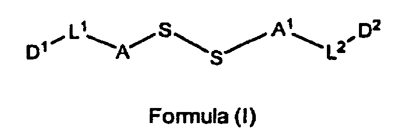 wherein,
A and A' independently represent a bond, (CH₂)_{d}, 1,2-phenylene, 1,3-phenylene or 1,4-phenylene;
d is 1-8;
D¹ and D² are independently selected from: cetirizine, desloratidine, terfenadine, fexofenadine and pseudoephedrine;
L¹ and L² independently represent a bond, O, S, NR¹, L, or a linkage selected from the group consisting of: 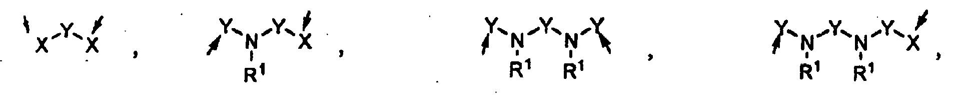 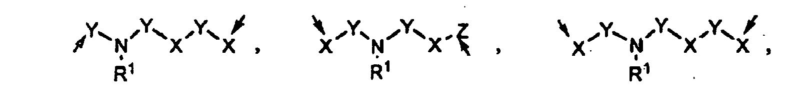 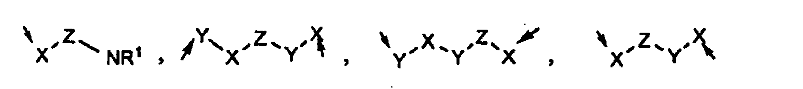
L is a group with bonding in any direction, independently selected from the group consisting of: 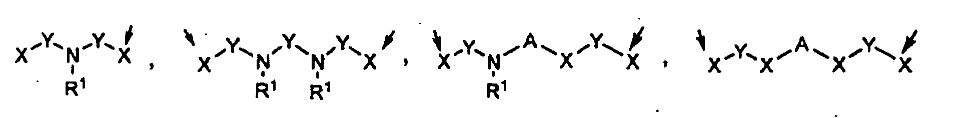 and 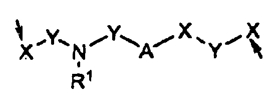 where
   X independently represents a bond, C, O, S, or NR¹;
   Y independently represents a bond, C=O, C=S, S=O, SO₂, P(=O)XR¹, or (CH₂)_{d}; wherein
   d is as defined;
   Z independently represents a bond, or (CH₂)ⱼ; wherein, j is 1-4;
   R¹ independently represents a bond, H, (C₁-C₈)alkyl, substituted(C₁-C₈)alkyl, (C₅-C₁₄)aryl, aralkyl or M^{e+};
   M independently represents Na, K or a pharmaceutically acceptable metal ion; and
   e = 1-3.

In one preferred embodiment, L² is O; A and A¹ are independently (CH₂)_{d}, 1,2-phenylene, 1,3-phenylene, or 1,4-phenylene; and d is 1-8.

In another preferred embodiment A and A' are (CH₂)₂ and D² is the same as D¹.

In a further preferred embodiment D² is the same as D¹.

In another preferred embodiment the compound is selected from: 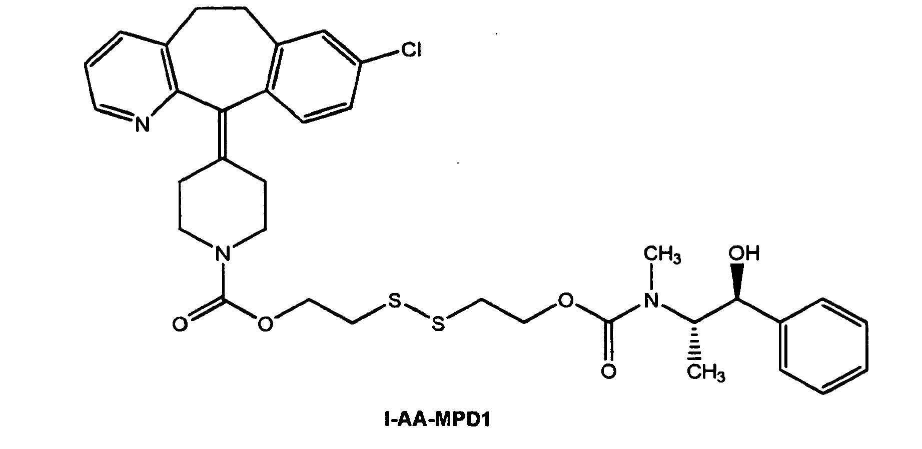 and

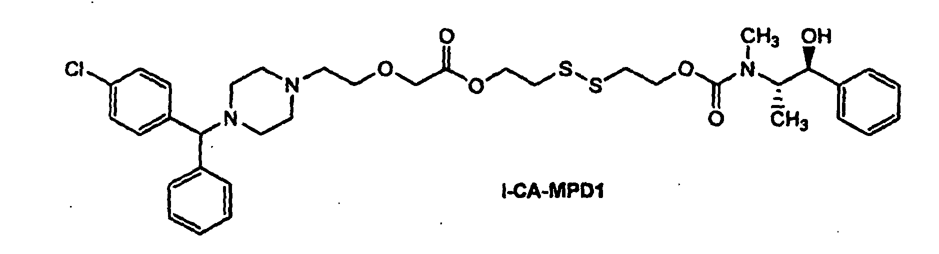

In another preferred embodiment the compound is selected from I-CA-MPD2, I-CA-MPD3, I-CA-MPD4 and I-AH-MPD16 or pharmaceutically acceptable salts thereof.

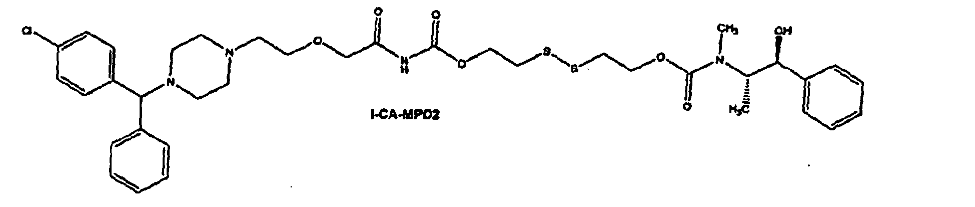

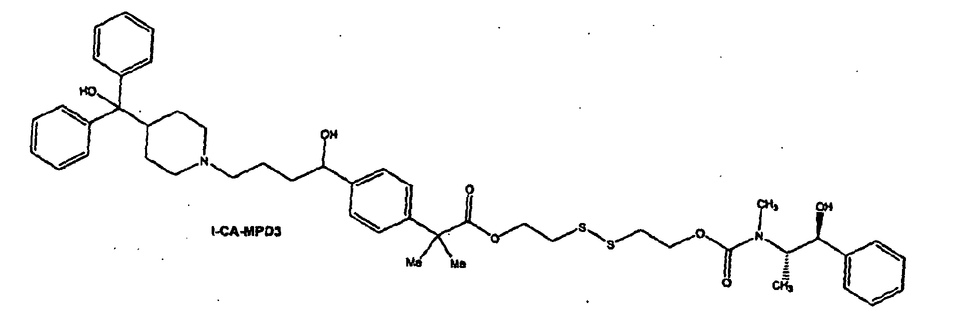

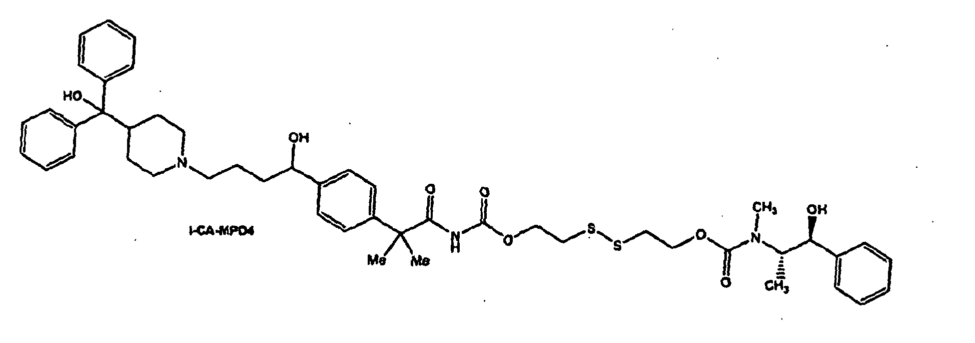

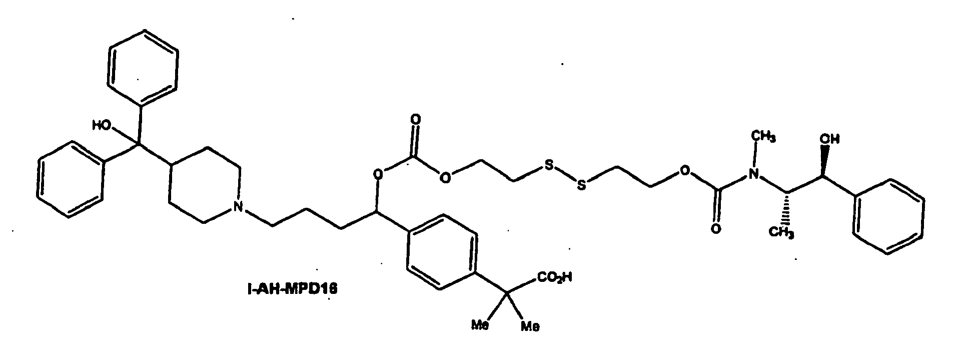

A further embodiment of the invention provides a pharmaceutical composition comprising a therapeutically effective amount of the compound as defined in the first aspect of the invention, or a pharmaceutical salt thereof, and one or more pharmaceutically acceptable carriers, vehicles or diluents. Another embodiment provides this composition for use in the treatment of diseases where a chronic, sustained and selective release of the constituent drug(s) is beneficial.

In preferred embodiments D¹ and D² as pairs of drugs are selected from: Pseudoephedrine and Fexofenadine/Cetinzine/Desloratadine.

Further embodiments of the the invention provide a compound as defined in the first aspect of the invention, for use in the treatment of diseases where a chronic, sustained and selective release of the constituent drug is beneficial.

A second aspect of the invention provides intermediates obtained in the preparation of compounds of the first aspect of the invention, wherein the intermediates are selected from:

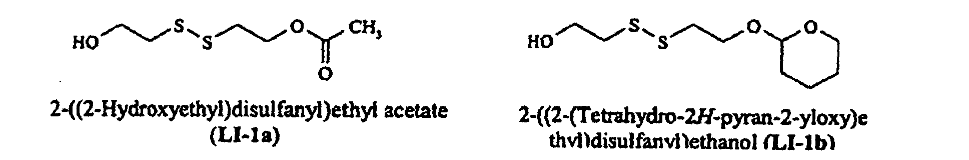

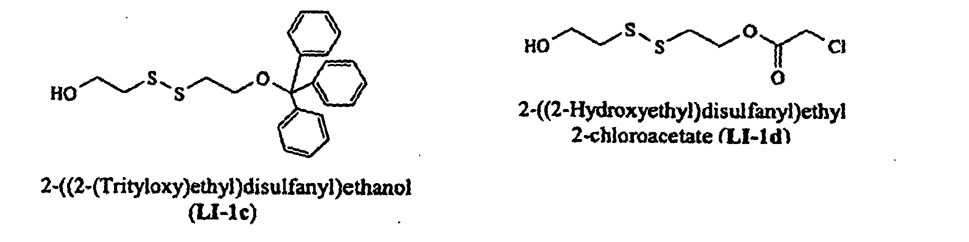

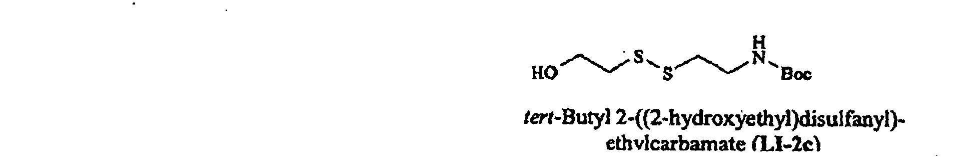

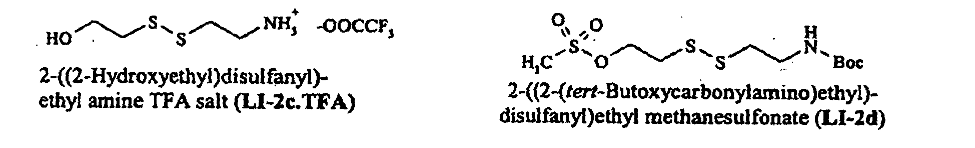

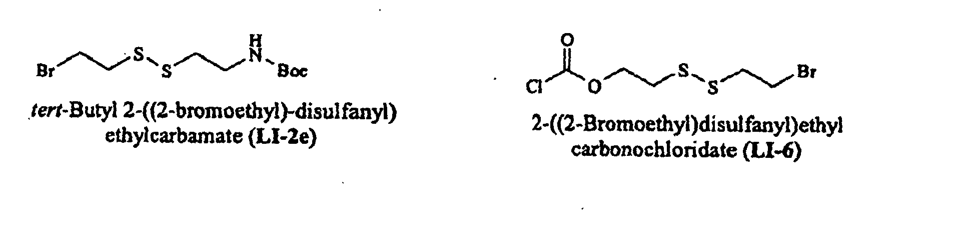

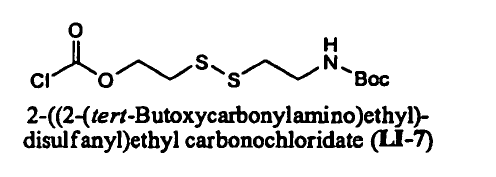

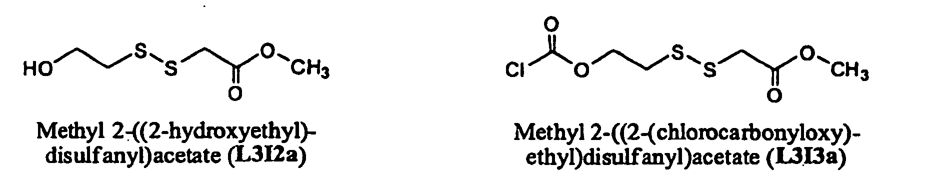

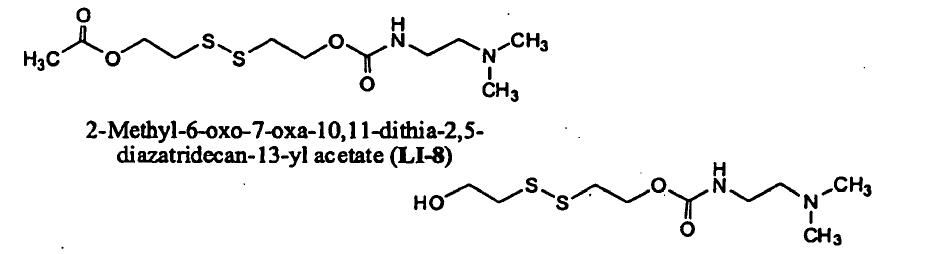

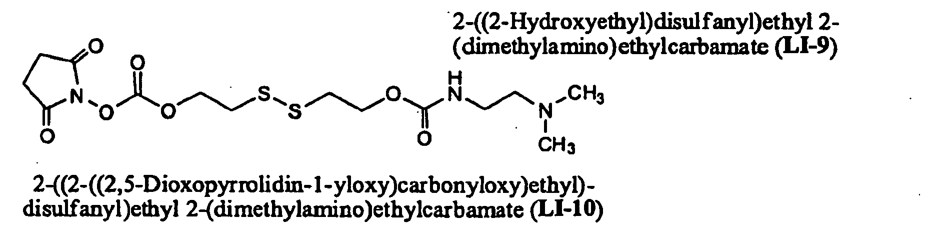

According to a third aspect of the invention there is provided a process for the preparation of the compounds of the first aspect of the invention, wherein the preparation of said compounds involves use of the intermediates selected from a group consisting of :

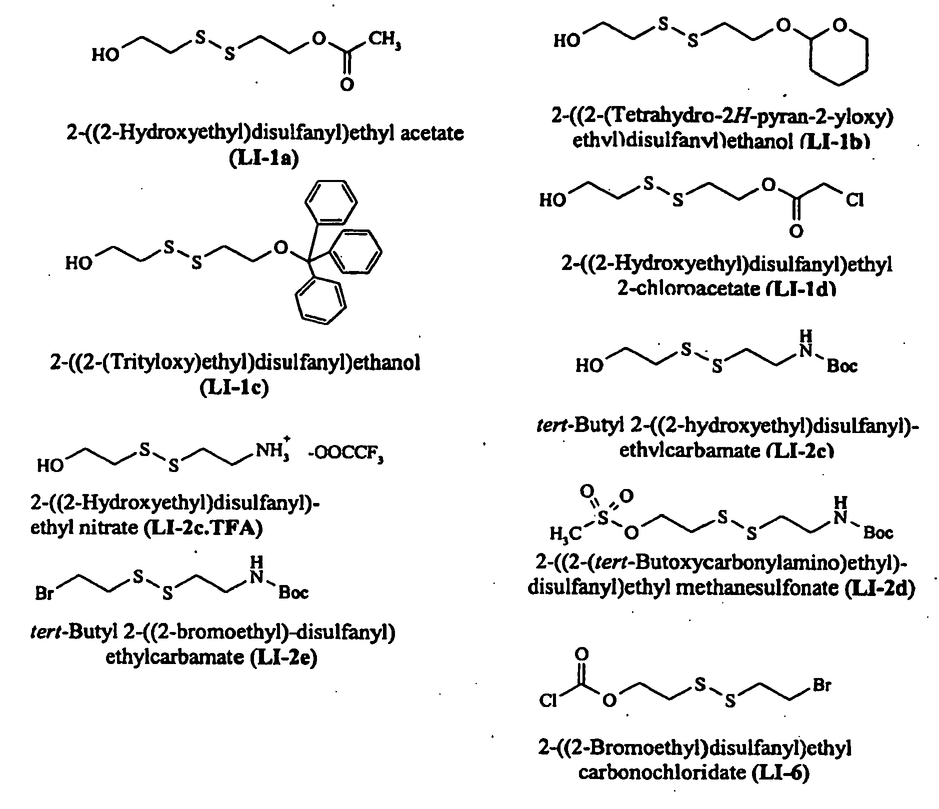

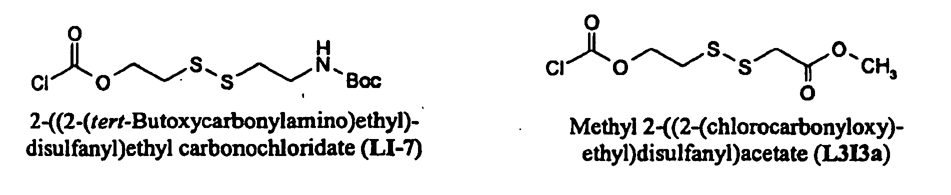

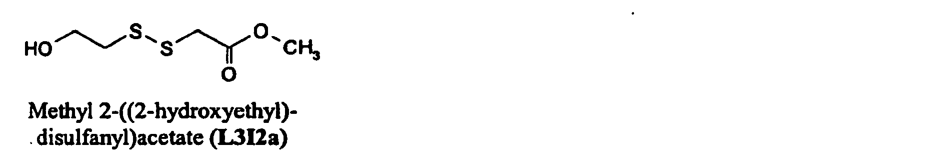

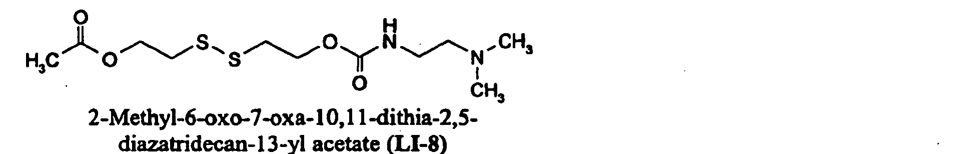

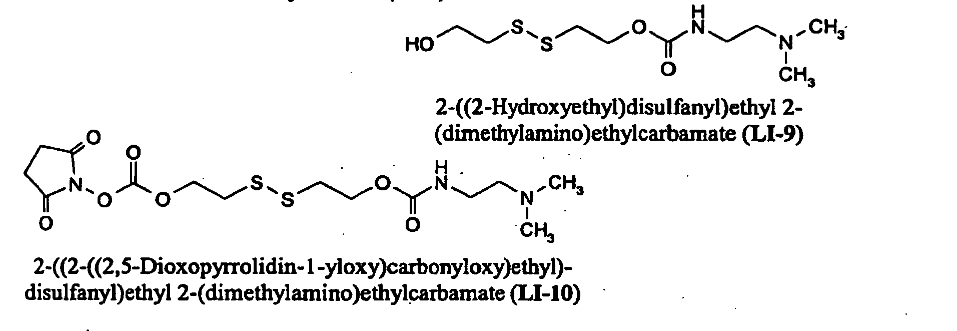

According to a fourth aspect of the invention there is provided a process for the preparation of a compound of the first aspect of the invention, or a pharmaceutically acceptable salt, thereof, wherein the process is selected from:
Process 1:
   A ) Monoprotection of Bis-(2-hydroxyethyl)disulphide (SL-1) with an appropriate hydroxyl protecting group to give the corresponding monoprotected intermediate LI-1x, 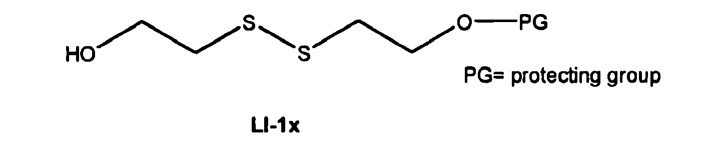
   B) Converting the monoprotected intermediate LI-1x obtained in step A to an activated formyl intermediate LI-1xy by treating with phosgene or its equivalent,
      and
   C) Reacting the activated formyl intermediate LI-1xy obtained in the step B with an appropriate amino- or hydroxyl containing drug (D¹) to give the corresponding compound of formula I;
Process 2:
   A) Converting carboxyl containing drug (D¹) into its activated acyl halide or imidazolide or isocyanate by known methods, and
   B) Reacting the intermediate obtained in the step A with the monoprotected linker intermediate LI-1x to obtain the compound of formula I;
      or
Process 3:
   A) Monoprotection of Bis-(2-hydroxyethyl)disulphide (SL-1) with an appropriate hydroxyl protecting group to give the corresponding monoprotected intermediate LI-1x,
   B) Reacting formyl linker intermediate LI-1xy with amino or hydroxyl containing drug (D¹) to obtain the prodrug of formula I with free hydroxyl group on the linker,
   C) Converting the intermediate obtained in the step B into activated formyl halide or imidazolide derivative, and
   D) Reacting the intermediate obtained in the step C with the drug D² to obtain the mutual prodrug of formula I.

### Detailed Description of the Invention

The present invention relates to compositions comprising the compounds of Formula I methods of preparation of prodrugs, including mutual prodrugs, and double prodrugs comprising the compounds of Formula I.

The compounds of the present invention are prodrugs or mutual prodrugs in which known therapeutic agents or potential therapeutic agents are linked covalently to novel biocleavable linkers.

Unless otherwise defined, all technical and scientific terms used herein have the same meaning as commonly understood by one of ordinary skill in the art to which this invention belongs. Listed below are definitions of various terms used to describe the compounds of the present invention. These definitions apply to the term as they are used throughout the specification (unless they are otherwise limited in specific instances) either individually or part of a larger group.

The term "amino-containing" refers to drug/carrier molecule with NH functional groups such as amino (both primary and secondary), amide, urea, sulfonamide, carbamate, phosphoramadite, sulfamate, hydrazone, semicarbazone, thiosemicarbazone, hydrazide and carbazate. This also includes NH-containing heterocylic compounds such as imidazoles, benzimidazoles, pyrazoles, benzpyrazols, pyrrols, indoles, triazoles, tetrazoles, benzotriazoles, benzotetrazoles and their derivatives. These NH-containing heterocyclic compounds can be sub-structures of more complex drug/carrier molecules. Amino group of the candidate drug can be primary or secondary (both acyclic and cyclic) which include amide-NH, sulfonamide-NH, carbamate-NH, sulfamate-NH, hydrazide-NH, hydrazone-NH, semicarbazone-NH, thiosemicarbazone-NH, urea-NH and also drugs containing indole, imidazole, benzimidazole, thiazole oxozole, pyrrole, pyrazole, triazole, tetrazole, or similar NH-containing heterocylic sub-structures of a more complex drug molecule.

The term "hydroxyl-containing" refers to drug/carrier molecules with hydroxyl groups (primary, secondary and tertiary) including hydroxyl groups of hydroxamic acids and ketoximes derived from keto-containing molecules. Hydroxyl group of drugs can be of primary, secondary or tertiary nature.

The term "sulfahydryl-containing" refers to drug/carrier with free sulfahydryl (SH) group.

The term "halo" refers to fluoro, chloro, bromo, and iodo.

The term "halide" refers to fluoride, chloride, bromide, and iodide.

The term "alkyl" refers to acyclic alkyl chains. For example, the term "C₁-C₈ alkyl" refers to methyl, ethyl, propyl, isopropyl, butyl, s-butyl, t-butyl, pentyl, hexyl, heptyl and octyl.

The term "cycloalkyl" refers to cyclic alkyl chains, e.g., the term "C₃-C₈ cycloalkyl" refers to cyclopropyl, cyclobutyl, cyclopentyl, cyclohexyl, cycloheptyl and cyclooctyl.

The term "aryl" refers to phenyl and naphthyl.

The term "aralkyl" refers to benzyl and phenethyl.

The term "alkoxy" refers to both acyclic and cyclic C₁-C₈ alkyloxy. For example, the term "C₁-C₈ alkyloxy" refers to methoxy, ethoxy, propoxy, isopropoxy, cyclopropoxy, butoxy, cyclobutoxy, s-butoxy, t-butoxy, cyclopentyloxy, pentyloxy, hexyloxy, cyclohexyloxy, heptyloxy, cycloheptyloxy, octyloxy and cyclooctyloxy.

The term "heterocyclic" and "heteroaryl" refers to both saturated and unsaturated 5- and 6-membered rings (including benzo-fused) containing from 1 to 4 heteroatoms selected from the group consisting of nitrogen, oxygen and sulfur. All of these rings may be substituted with up to three substituents independently selected from the group consisting of amino, halo, alkoxy, alkyl, cyano, nitro, hydroxyl, sulfahydryl and carboxyl, Saturated rings include, for example, pyrrolidinyl, piperidinyl, piperazinyl, tetrahydrofuryl, oxazolidinyl, dioxanyl and pyranyl. Benzofused saturated rings include indolinyl, 1,2,3,4-tetrahydroquinolinyl and 1,2,3,4-tetrahydroisoquinolinyl. Unsaturated rings include furyl, thienyl, pyridinyl, pyrrolyl, N-methylpyrrolyl, oxazolyl, isoxazolyl, pyrazolyl, imidazolyl, tetrazolyl, triazolyl, oxadiazolyl, thiadiazolyl, thiazolyl, pyrimidinyl, pyrazinyl and pyridazinyl. Benzofused unsaturated rings include isoquinolinyl, benzoxazolyl, benzthiazolyl, quinolinyl, benzofuranyl, thionaphthyl and indolyl.

The term "substituted alkyl" refers to acylic and cyclic alkyl groups substituted with one or more of groups such as alkyl, aryl, hydroxy, alkoxy, cyano, carboxyl, sulfahydryl, alkylthio, amino, nitro, halo, carbonyl, carbamato, sulfamato, sulfonato and sulfato.

The term "substituted aryl" refers to aryl groups substituted (including fused) with one or more of groups such as alkyl, aryl, hydroxy, alkoxy, cyano, carboxyl, sulfahydryl, alkylthio, amino, nitro, halo, carbonyl, carbamato, sulfamato, sulfonato and sulfato.

The term "amino acid" refers to molecules containing one or more amino and carboxyl groups. Examples of alfa-amino acids (D-, L- and DL- amino acids) -include natural alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine. Other examples include beta-amino acids and known unnatural amino acids.

The term "amino acid ester" as used in this specification refers to an amino acid where the carboxyl group is substituted with a C₁-C₈ alkyl group. That is, the alkyl group when taken together with the carboxyl group forms a C₁ -C₆ alkyl ester. It is appreciated that some amino acids (e.g., aspartic acid and glutamic acid) have two carboxyl groups these may form mono- and di-esters.

The term "protecting group" (PG) refers to an 'amino protecting group' or a 'hydroxyl protecting group' or a 'carboxyl protecting group'.

The term "amino protecting group" refers to a group that selectively blocks or protects the amino functionality in presence of other functional groups on the molecule. Examples of such amino-protecting groups include the formyl group, the trityl group, the phthalimido group, the acetyl group, the trifluoroacetyl group, the chloroacetyl, bromoacetyl, and iodoacetyl groups, urethane-type blocking groups such as benzyloxycarbonyl ("CBZ"), 9-fluorenylmethoxycarbonyl ("FMOC"), tert-butoxycarbonyl ("BOC") and trichloroethylcarbonyl. Additional examples of amino protecting groups are described by T. W. Greene, "Protective Groups in Organic Synthesis", John Wiley and Sons, New York, N.Y., 1991. Molecules with two or more amino groups may form mono-, di-, tri-, poly-, protected derivatives depending on the reaction conditions used.

The term "hydroxyl protecting group" refers to a group that selectively blocks or protects hydroxyl functionality in presence of other reactive functional groups on the molecule. Examples of such hydroxyl-protecting groups include, for example, ether groups including methyl and substituted methyl ether groups such as methyl ether, methoxymethyl ether, methylthiomethyl ether, tert-buylthiomethyl ether, triphenylmethyl tetrahydropuranyl (THP), (phenyldimethylsilyl)methoxy-methyl ether, benzyloxymethyl ether, p-methoxybenzyloxy-methyl ether, and tert-butoxymethyl ether; substituted ethyl ether groups such as ethoxyethyl ether, 1-(2-chloroethoxy)-ethyl ether, 2,2,2-trichloroethoxymethyl ether, and 2-(trimethylsilyl)ethyl ether, isopropyl ether groups; phenyl and substituted phenyl ether groups such as phenyl ether, p-chlorophenyl ether, p-methoxyphenyl ether, and 2,4-dinitrophenyl ether, benzyl and substituted benzyl ether groups such as benzyl ether, p-methoxybenzyl ether, o-nitrobenzyl ether, and 2,6-dichlorobenzyl ether; and alkylsilyl ether groups such as trimethyl-, triethyl- and triisopropylsilyl ethers, mixed alkylsilyl ether groups such as dimethylisopropylsilyl ether, tert-butyldimethylsilyl ether and diethylisopropylsilyl ether, and ester protecting groups such as acetate ester, formate ester, benzylformate ester, mono-, di-, and trichloroacetate esters, pivalate ester, phenoxyacetate ester, and p-chlorophenoxyacetate, benzyloxycarbonate, 9-fluorenylmethoxycarbonate, tert-butoxycarbonate, trichloroethylcarbonate, carbamate and sulfamate. Additional examples of hydroxyl protecting groups are described by T. W. Greene, "Protective Groups in Organic Synthesis", John Wiley and Sons, New York, N.Y., 1991. Molecules with two or more hydroxyl groups may form mono- and di-esters/ethers depending on the reaction condition.

The term "carboxyl protecting group" refers to a group that selectively blocks or protects carboxyl functionality in presence of other reactive functional groups on the molecule. Examples of such carboxyl-protecting groups include, for example (substituted) alkyl esters such methyl ester, ethyl ester, t-butyl ester, (substituted) benzyl ester and trichloroethyl ester. Additional examples of carboxylic acid protecting groups are described T. W. Greene, "Protective Groups in Organic Synthesis", John Wiley and Sons, New York, N.Y., 1991. Molecules with two or more carboxylic acid groups may form mono-, di-, tri-, tetra-, poly- protected derivatives depending upon the reaction conditions used.

The term "carbonyl activating group" refers to leaving group ("LG") of a carboxyl derivative that is easily replaced by an incoming nucleophile. Such "LG" groups include (substituted) alkoxy, aryloxy, nitrogen containing unsaturated heterocycles such as N-oxybenzotriazole, imidazolyl, o/p-nitrophenoxy, pentachlorophenoxy, N-oxysuccinimide, N,N-dicyclohexylisoure-O-yl, N-hydroxy-N-methoxyamino, acetates, formates, sulfonates such as methanesulfonate, ethanesulfonate, benzenesulfonate, or p-toluenesulfonate; and halides especially fluoride, chloride, bromide, or iodide.

The term "carbonyl activating reagent" refers to a reagent that converts the carbonyl of a carboxylic acid group into one that is more susceptible to nucleophilic attack and includes such reagents as those found in "The Peptides", Gross and Meienhofer, Eds., Academic Press (1979), Ch. 2, and M. Bodanszky, "Principles of Peptide Synthesis", 2.sup.nd Ed., Springer-Verlag Berlin Heidelberg, 1993, hereafter referred to as "The Peptides" and "Peptide Synthesis" respectively. Carbonyl group (i.e., aldehyde or keto group) of candidate drugs may be converted first to aldoxime, ketoxime, hydrazone or semicarbazone, before coupling to the linker. Specifically, carbonyl activating reagents include thionyl bromide, thionyl chloride and oxalyl chloride esters of alcohols such as nitrophenol and pentachlorophenol; and compounds such as 1,1'-carbonyldiimidazole (CDI), benzotriazole, imidazole, N-hydroxysuccinimide, dicyclohexylcarbodiimide (DCC), EDC, phosgene or its equivalents and N, N-dimethylaminopyridine (DMAP).

The terms "phosgene or its equivalents" refer to phosgene or it equivalents such as diphosgene, triphosgene, CDI, DSC, BTBC, alkoxycarbonyl chlorides and o/p-nitrosubstituted phenoxycarbonyl chlorides.

In general, the term "pharmaceutical" when used as an adjective means substantially non-toxic to living organisms.

The terms "pharmaceutically acceptable metal ions or salts" refer to salts of the compounds of this invention, which are substantially non-toxic to living organisms. See, e.g., Berge, S. M. et al., "Pharmaceutical Salts", J. Pharm. Sci., 66:1, 1977. Typical pharmaceutical salts include those salts prepared by reaction of the compounds of this invention with an inorganic or organic acid or base. Such salts are known as acid addition or base addition salts respectively. These pharmaceutical salts frequently have enhanced solubility characteristics compared to the compound from which they are derived, and thus are often more amenable to formulation as liquids or emulsions. Examples of pharmaceutically acceptable salts are those with inorganic bases such as sodium, potassium, calcium, magnesium, and hydroxides, or with organic bases such as lysine, arginine, triethylamine, dibenzylamine and piperidine.

The term "suitable solvent" refers to a solvent that is inert to the ongoing reaction and sufficiently solubilizes the reactants to effect the desired reaction. Examples of suitable solvents include dichloromethane, chloroform, 1,2-dichloroethane, diethyl ether, tert-butylmethyl ether, acetonitrile, ethyl acetate, 1,3-dimethyl-2-imidazolidinone, tetrahydrofuran, dimethylformamide, benzene, toluene, xylene, N-dimethylacetamide, N-methylpyrrolidine, chlorobenzene, dimethylsulfoxide, dimethoxyethane, water, methanol, ethanol, isopropanol, pyridine, nitromethane and mixtures thereof.

The term "suitable base" refers to a base, which acts as a proton trap for any protons, which may be produced as a byproduct of the desired reaction, or to a base, which provides a reversible deprotonation of an acidic proton from the substrate and is reactive enough to effect the desired reaction without significantly effecting any undesired reactions. Examples of such bases include, carbonates, bicarbonates, and hydroxides (e.g., lithium, sodium, potassium, magnesium, calcium and the like), sodium/potassium/calcium hydride, sodium/potassium alkoxide (i.e., methoxide, ethoxide, tert-butoxide and the like), triethylamine, diisopropylethylamine, N-methylpyrrolidine, N-methylmorpholine, tetramethylguinidine, or aromatic nitrogen containing heterocycles such as pyridine and 4-(dimethylamino)pyridine (DMAP).

The term "therapeutic agent" refers to biologically active molecules such as drugs, vitamins, and other molecules, agents or substances concerned with or contributing to the treatment and cure of illness or contributing to the general well being of a mammal or human. The therapeutic agents can be both known and investigational drugs compiled in drug databases such as the Merck Index, IDdb, Prous Science's Integrity^{®}, Prous Science Drugs of the Future^{™}, The Ensemble^{®} and the like. The Merck Index is a one-volume encyclopedia of chemicals, drugs and biologicals that contains more than 10,000 monographs. Each monograph in this authoritative reference source is a concise description of a single substance or a small group of closely related compounds. Prous Science is an international health science publishing company, established in 1958 and headquartered in Barcelona, Spain. Prous Science Drugs of the Future™, produced by Prous Science Publishers, contains comprehensive drug monographs providing product information on new compounds, including the synthesis and corresponding schemes, pharmacological action, pharmacokinetics and metabolism, toxicity, clinical studies, manufacturer, and references. Information on compounds is continuously updated as advances in development status are disclosed worldwide. The Prous Science Integrity^{™} is a drug R&D portal where knowledge areas are coordinated to provide a harmonious and interrelated whole, which includes Drugs & Biologics, Targets, Organic Synthesis, Experimental Pharmacology, Pharmacokinetics and Metabolism, Clinical Studies, Disease Briefings, Companies & Markets, Literature and Patents. The *Investigational Drugs database* (*IDdb*), developed by Thomson Current Drugs, is a pharmaceutical competitor intelligence service. It covers all aspects of investigational drug development, from first patent to eventual launch or discontinuation. The Ensemble® on the Web provides essential information, including chemical structures, on more than 140,000 compounds with demonstrated biological activity in the drug research and development pipeline.

One aspect of the invention is to provide mutual prodrugs of two therapeutic agents currently used for use in combination therapy utilizing novel bio-cleavable linkers, water-soluble prodrugs of insoluble and sparingly-soluble therapeutic agents using the same linker technology, and water-soluble double prodrugs of sparingly-soluble therapeutic agents using the same linker technology.

The candidate drugs are selected from cetirizine, desloratidine, terfenadine, fexofenadine and pseudoephedrine.

The compounds of the invention, which have one or more asymmetric carbon atoms, can exist as the optically pure enantiomers, pure diastereomers, enantiomer racemic mixtures, diastereomer racemic mixtures, racemates or racemate mixtures. Within the scope of the invention are also all the possible, stereoisomers and their mixtures of the compounds of formula **(I).**

### POTENTIAL EXAMPLES OF MUTUAL PRODRUGS/CODRUGS:

Mutual prodrugs made from an amino-containing therapeutic agent and another amino-containing therapeutic agent:
A Mutual Prodrug of desloratadine and pseudoephedrine (I-AA-MPD1) is proposed as a potential treatment option for seasonal allergic rhinitis (SAR). Desloratadine (an active metabolite of loratadine) is a new, non-sedating, long-acting histamine antagonist and has been shown effective in the treatment of nasal and non-nasal symptoms associated with SAR. Pseudoephedrine is an oral decongestant.

### Mutual prodrugs made from an amino-containing therapeutic agent and a carboxyl-containing therapeutic agent:

A mutual prodrug of cetirizine and pseudoephedrine (I-CA-MPD1) is proposed for treatment of rhinitis. Cetirizine is an antihistamine and pseudoephedrine is a nasal decongestant.

### Plausible Mechanisms of Drug Release from Prodrugs

Drugs can be released from the prodrugs and mutual prodrugs via cleavage of bio-labile linker(s) in vivo (cleavage can be either chemical or enzymatic or both) by illustrative mechanisms as shown in Schemes M2 through M5.

Plausible mechanisms of drug release from mutual prodrugs of one carboxyl-containing and one amino-/hydroxyl-containing drug is shown in Scheme M2.

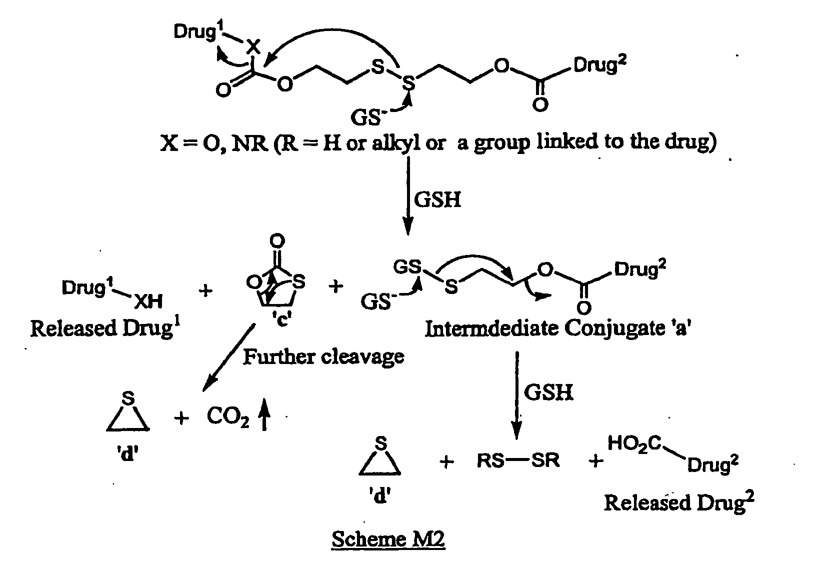

Plausible mechanism of drug release from prodrugs (including mutual prodrugs of amino-, hydroxyl-and carboxyl-containing drugs) containing modified bio-labile linkers is shown in Scheme M3. Thus, the thiolate anion derived from the attack of glutathione on disulfide of the prodrug may trigger cyclization to release the free drug (D1-X^{m1}H) and a stable six-membered (or five-membered, if X^{m2} is a bond) thio-lactone intermediate.

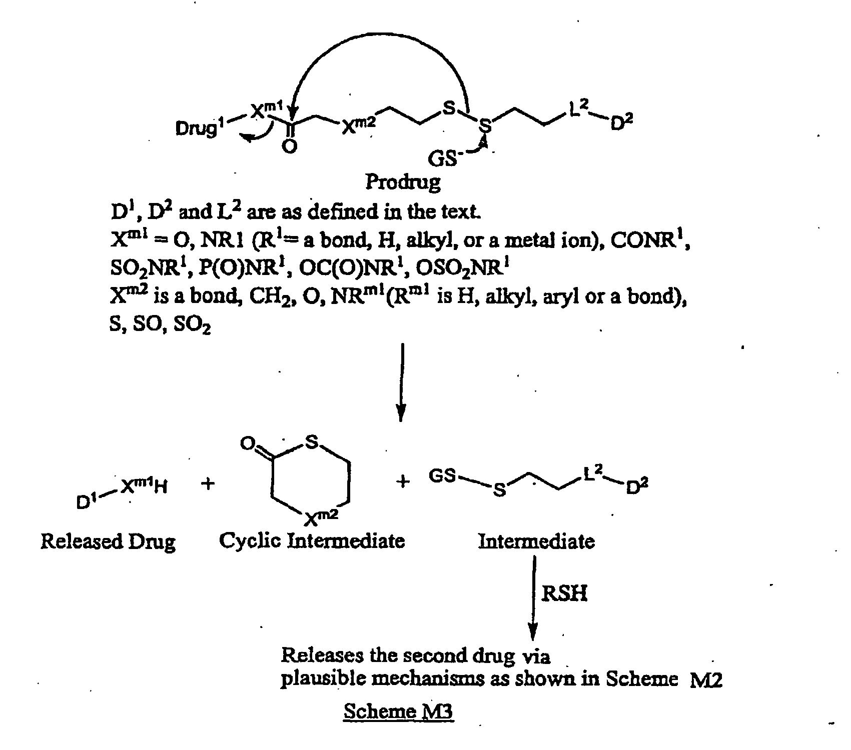

Plausible mechanisms of drug release from double/mutual prodrugs containing additional linkages to couple two hydroxyl-containing drugs are shown in Scheme M4. Thus, the thiolate anion generated by the attack of glutathione on disulfide bond of the prodrug triggers further cleavage as shown to release the free drug (D¹-OH) and a five-membered 2-imidazolidone. Through in vitro decomposition studies, we have found that the drug release from this type of prodrug is more facile when R group is an alkyl group.

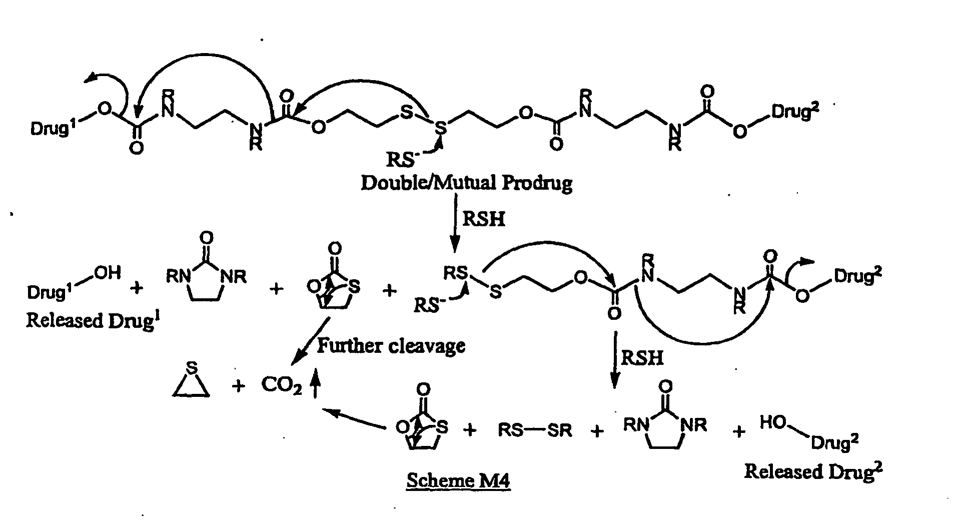

This invention also covers novel bio-labile linkers containing 1,4-phenylene group and 1,2-phenylene group as shown in Schemes M5 and M6, respectively. As depicted in Scheme M5, the linker is expected to release the free Drug¹ upon glutathione-assisted cleavage and may generate 1,4-quinonemethid (ea) as a byproduct via 1,6-elimination process. Similarly, the free Drug² is expected to be released from the intermediate conjugate (a) as shown in the scheme.

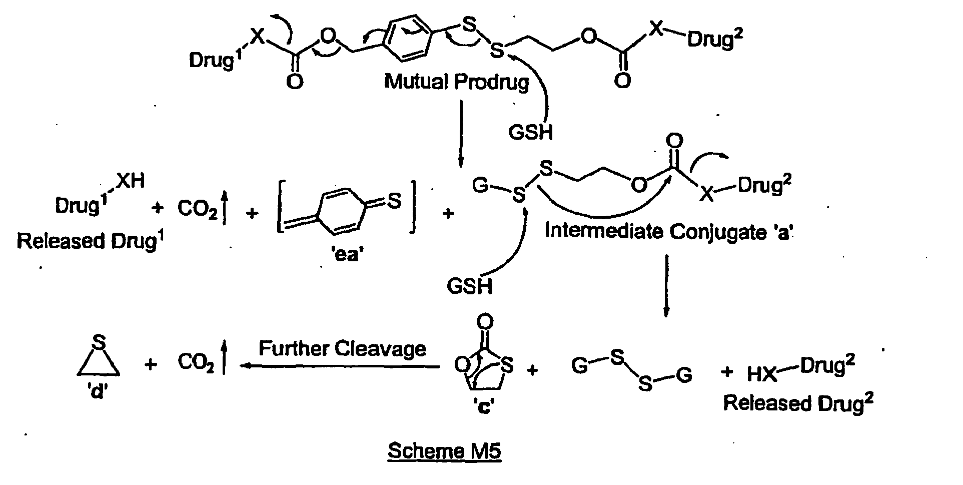

As depicted in Scheme M6; the 1,2-phenylene-containing linker is also expected to release free drugs upon glutathione assisted cleavage and generate 1,2-quinonemethid (eb) as a byproduct via 1,4-elimination process (via pathway 'b'). However, this linker can also cleave via pathway 'a' to generate benzo-monothiocarbonate as a byproduct. Although the generated byproducts seem to be toxic, they are likely to be quickly neutralized by detoxification enzymes in the body.

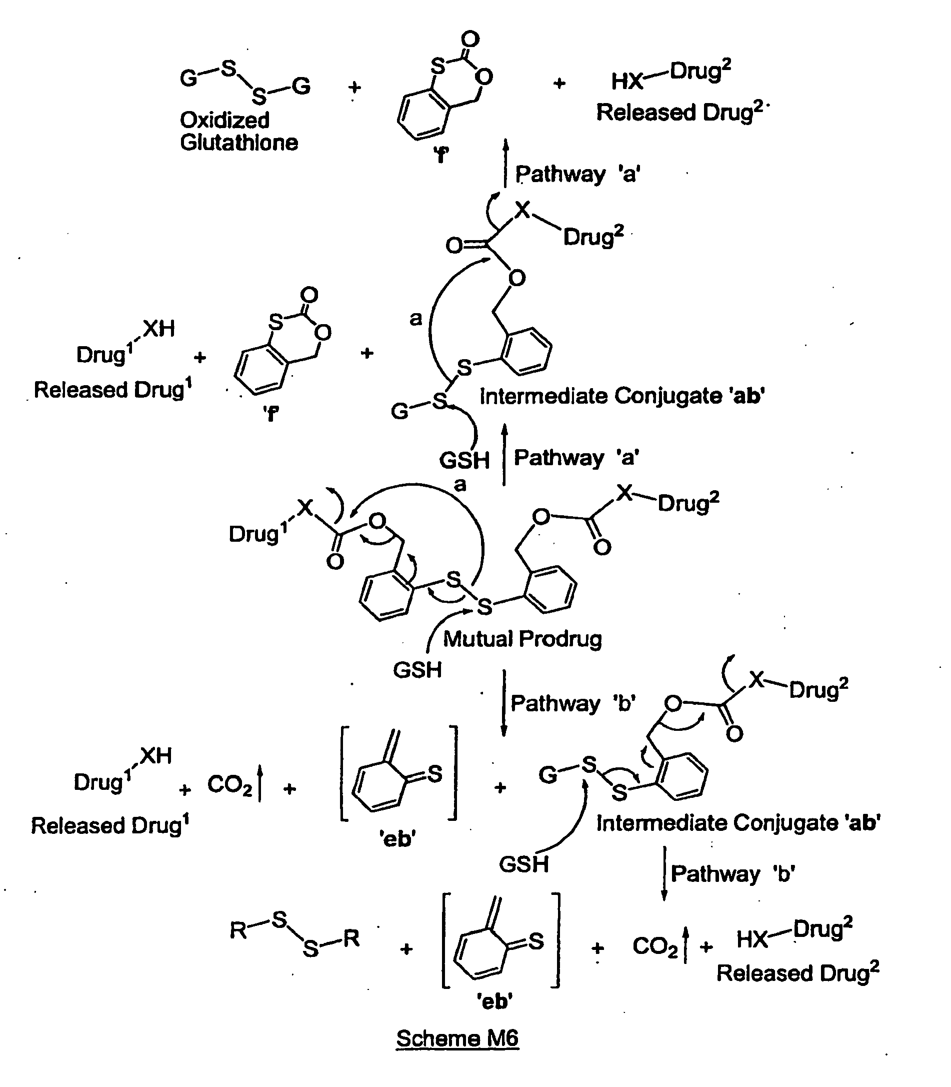

Where GSH is glutathione (reduced) or any other in vivo bioreductive agent that can reduce the disulfide bond. As illustrated, cleavage of disulfide bond triggers further breakage of the remaining portion of the linker to release the free drugs. In the process, some byproducts are generated and these are either eliminated or further degraded by some biological process. For clarity, the mechanism of cleavage of the linker is shown as occurring in stepwise manner. However, both the steps can possibly occur in a concomitant fashion to release both the drugs simultaneously.

As illustrated in Scheme M3 and M4, Linkers may have additional spacer groups between one side (or both sides) of the linkers and the drug molecule and some of these spacer groups may be cleaved independently by a chemical or enzymatic process to release the drugs prematurely before the cleavage of disulfide linkage. The prodrugs and mutual prodrugs containing such spacer groups may be useful when faster release of drug(s) is desired.

### EXPERIMENTAL

### ABBREVIATIONS USED:

- BOP:: Benzotriazol-1-yl-oxy-*tris*(dimethylamino)phosphonium hexafluorophosphate
- DMF:: N,N-Dimethylformamide
- DSC:: N,N'-Disuccinimidyl carbonate
- CDI:: N,N'-Carbonyldiimidazole
- DTE:: Dithioerythritol
- DTT:: Dithiothreitol
- DCC:: N,N'-Dicyclohexylcarbodiimide
- EDAC. HCl:: 1-Ethyl-(3-dimethylaminopropyl)carbodiimide hydrochloride
- HBTU:: O-(Benzotriazol-1-yl)-N,N,N',N'-tetramethyluronium hexafluorophosphate
- TUTU:: O-(Benzobiazol-1-yl)-N,N,N',N'-tetramethyluronium tetrafluoroborate
- EtOH:: Ethanol
- Et₂O:: Diethyl ether
- THF:: Tetrahydrofuran
- DMSO:: Dimethyl sulfoxide
- TEA:: Triethylamine
- DIPEA:: N,N-Diisopropylethylamine
- DCM:: Dichloromethane
- EtOAc:: Ethyl acetate
- DME:: Dimethoxyethane
- MeOH:: Methanol
- PE:: Petroleum ether
- RT:: Room temperature
- TFA:: Trifluoroacetic acid
- HOBT:: N-Hydroxybenzotriazole

### SYNTHETIC METHODS:

The prodrugs described herein can be prepared by any number of methods known/obvious to those skilled in the art. The synthetic approaches and the linkages are chosen depending upon the functional groups such as carboxyl, hydroxyl, amino or carbonyl groups present in the drug molecules to be used. The following illustrative methods, as shown in Schemes 1 through 8 can be utilized to make carbonate, urethane, amide, ester, N-acyl carbamate, N-acyl amide, N-acyl sulfamate, and N-acyl sulfonamide, N-acyl phosphoramide, N-oxycarbonylsulfonamide, N-oxycarbonylcarbamate linkages, etc., between drug(s) and linker(s).

### Method(s) of making carbonate linkage(s):

As depicted in the scheme 1, the carbonate linkage between the drug and the linker can be made by reacting the hydroxyl-containing drug (alternatively, hydroxyl group of the linker) with phosgene or its equivalents such as diphosgene, triphosgene, N,N'-carbonyldiimidazole (CDI), N,N'-disuccinimidyl carbonate (DSC) or 4-nitrophenyl chloroformate to give a reactive alkoxycarbonyl derivative, where LG is suitable leaving group such as a halide, imidazole, O-succinimide, 4-nitrophenoxide and the like, which can be reacted with hydroxyl group of the linker (alternatively, hydroxyl group of drug if the linker is converted to active alkoxycarbonyl derivative) in the presence of a suitable base and solvent

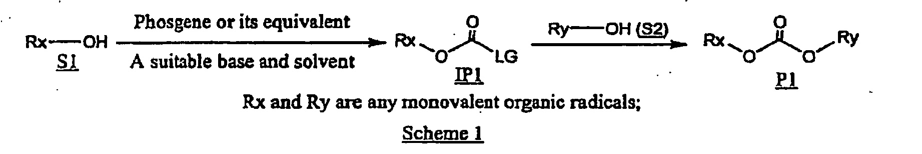

Bases such as triethylamine, diisopropylethylamine, 4-(dimethylamino)pyridine (DMAP), and the like, can be used. Suitable solvents include CH₂Cl₂, CHCl₃, DMF, THF, ACN, ethyl acetate and ethyl ether.

### Method(s) of making urethane linkage(s):

As shown in scheme 2, the urethane linkage between the drug and the linker can be made by reacting the hydroxyl-containing linker with phosgene or its equivalents (defined above) to give a reactive alkoxycarbonyl derivative, which can be reacted with amino-containing drug in the presence of a suitable base and solvent Alternatively, a urethane linkage can be made by adding an alcohol to an isocyanate.

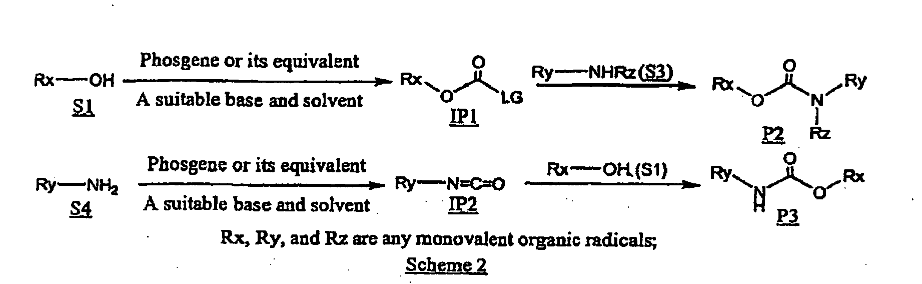

Suitable bases and solvents are same as defined above.

### Metbod(s) of making amide or ester linkage(s):

As shown in the Scheme 3, an amide or ester linkage between the drug and the linker can be made by reacting a carboxyl-containing drug with an amino- or hydroxyl-containing linker in the presence of a suitable coupling agent, base and solvent. Alternatively, the carboxyl-containing compound can be first converted to reactive carbonyl derivative such as an acid halide, a succinimide ester, a pentafluorophenyl ester, or an imidazolide, which can be treated with amino-containing or hydroxyl-containing linker in the presence of a suitable base and solvent to afford the corresponding amide or ester linkage(s), respectively (see, Bodanszky, M. and Bodanszky, A., The Practice of Peptide Synthesis, Springer-Verlag, New York, 1984)

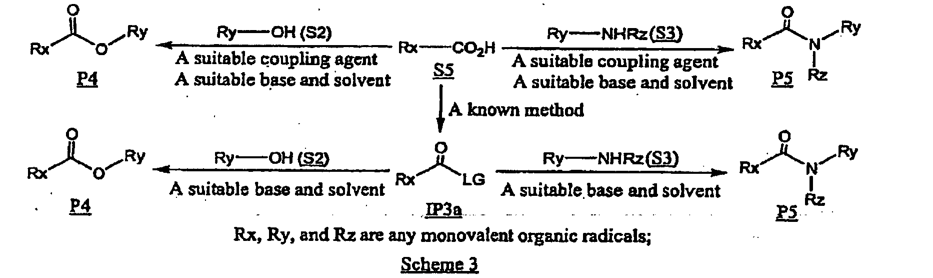

Suitable coupling agents include DCC, EDCI.HCl, BOP, HBTU, TBTU, DCC/HOBT, and EDC/HOBT. Suitable bases and solvents are same as defined above.

### Method(s) of making N-acyl carbamate and N-acyl urea linkage:

The linkage such as N-acyl carbamate linkage between the linker and drug can be made as shown in Scheme 4. Thus, treatment of an alcohol with phosgene or its equivalent can yield the corresponding carbonochloridate, which upon treatment with ammonia gas can give the corresponding carbamate intermediate. The carbamate nitrogen can be acylated by a suitable carboxylic acid derivatives such as anhydride or acid halide, a succinimide ester, a pentafluorophenyl ester or an imidazolide, in the presence of a suitable base to yield the corresponding N-acyl carbamate. Alternatively, N-acyl carbamate can be made by the reaction of an alcohol with N-acyl isocyanate, which can be prepared either by the reaction of the corresponding amide with oxalyl chloride (See, Speziale, A. J. et al., J. Org. Chem. 1962, 27, 3742; Speziale, A. J. et al., J. Org. Chem. 1963, 28, 1805-1811) or by the reaction of the corresponding acid chloride with silver cyanate. (See, Hill, AJ. et. al., J. Am. Chem. Soc., 1940, 62, 1595; Kim, D.K. J. Heterocyclic Chem. 1995, 32, 1625).

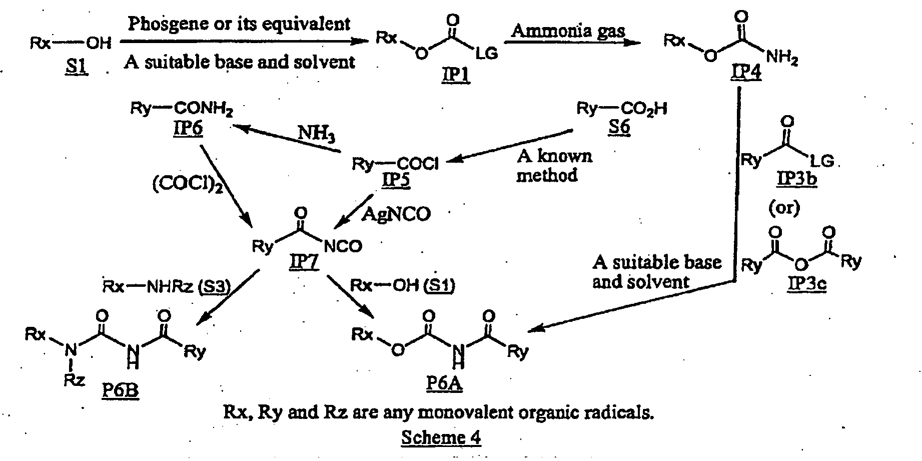

Suitable bases and solvents are same as defined above.

### Method(s) of making N-acyl amide linkage:

The N-acyl amide linkage between the linker and drug can be made as shown in Scheme 5. Thus, the amide nitrogen can be acylated by a suitable carboxylic acid derivatives such as anhydride or acid halide, a succinimide ester, a pentafluorophenyl ester or an imidazolide, in the presence of a suitable base to yield the corresponding N-acyl amide.

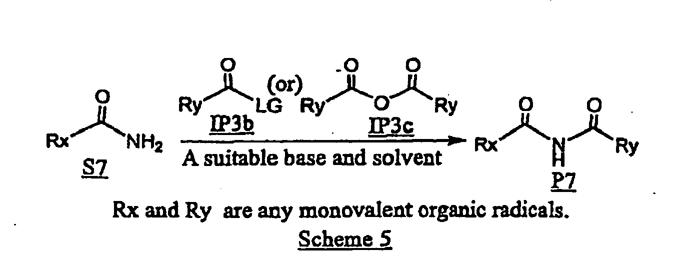

Suitable bases and solvents are same as defined above.

### Method(s) of making N-acyrl sulfamate linkage:

The linkage such as N-acyl sulfamate between the linker and drug can be made as shown in Scheme 6. Thus, treatment of an alcohol with sulfuryl chloride in the presence of suitable base gives the intermediate sulfochloridate, which can be converted to the corresponding sulfamate. Acylation of sulfamate nitrogen with a suitable carboxylic acid derivatives such as anhydride or acid halide, a succinimide ester, a pentafluorophenyl ester or an imidazolide, can yield the corresponding N-acyl sulfamate.

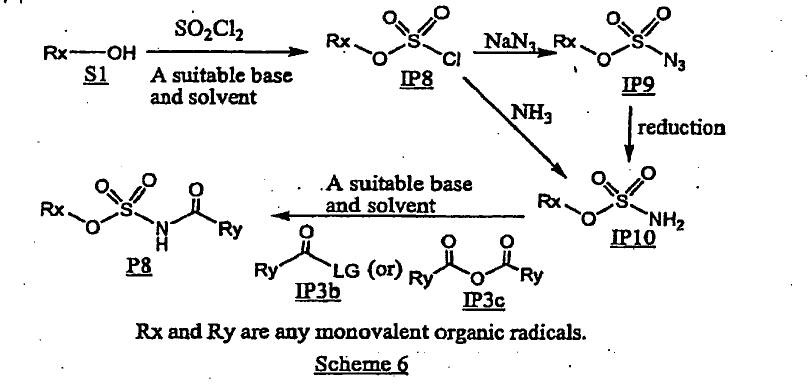

Suitable bases and solvents are same as defined above.

### Method(s) of making N-acyl/oxycarbonyl sulfonamide linkages:

The N-acyl/oxycarbonyl sulfonamide linkage between the linker and drug can be made as shown in Scheme 7. Thus, a sulfonamide nitrogen can be acylated by a suitable carboxylic acid derivatives such as anhydride or acid halide, a succinimide ester, a pentafluorophenyl ester or an imidazolide, to yield the corresponding N-acylsulfonamide, which can be metallated using an inorganic base. Similarly, the sulfonamide nitrogen can be acylated by a suitable formyl chloride derivative such as alkyloxycarbonyl chloride or imidazolide, to yield the corresponding N-alkyloxycarbonyl sulfonamide as shown in the scheme. Alternatively, the same linkage can be made by the reaction of an alcohol with sulfonyl isocyanate which can be prepared by known methods such by treatment of sulfonamide with oxalyl chloride (see, Hans Krzikalla et al., US2666787 or Smith, J. et al., J. Org. Chem. 1965, 30, 1260-1262) or by treatment of sulfonyl chloride with silver cyanate (See, Smith, J. et al., J. Org. Chem. 1965, 30, 1260-1262).

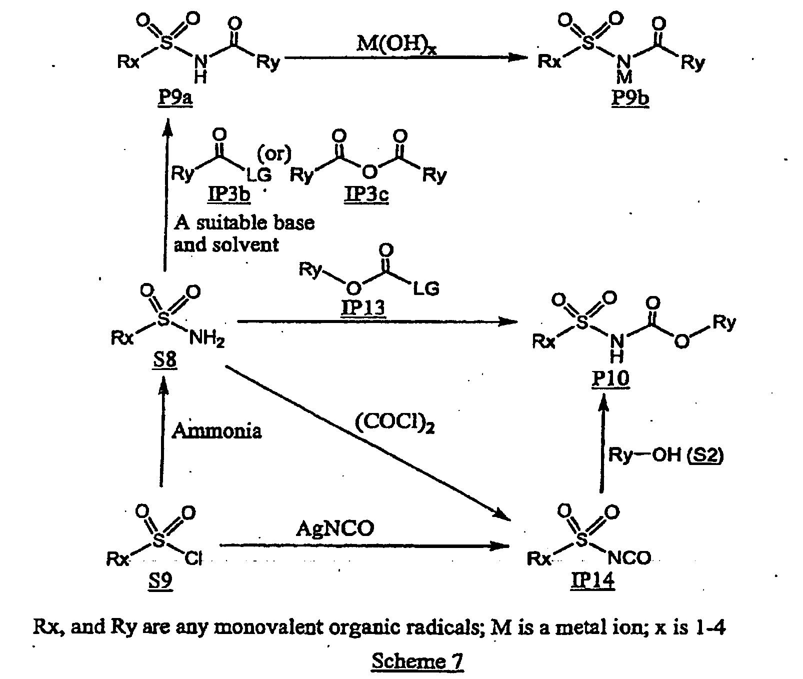

Suitable bases and solvents are same as defined above.

### Method(s) of making N-oxycarbonylcarbamate and N-oxycarbonylurea linkages:

The N-oxycarbonylcarbamate (or N-oxycarbonylurea) linkage between the linker and drug can be made as shown in Scheme 8. Thus, carbamate nitrogen can be acylated by suitable formyl chloride derivatives such as alkyloxycarbonyl chloride or imidazolide, to yield the corresponding N-alkyloxycarbonylcarbamate as shown in the scheme. Alternatively, the N-oxycarbonylcarbamate (or N-oxycarbonylurea) linkage between the linker and drug can be made by the reaction of an alcohol (or an amine) with carbamoyl isocyanate **(IP15A),** which can be prepared by know methods such by treatment of carbamate with oxalyl chloride (See, Grehn L, et al., Synthesis, 1988, 922-994) or by treatment of a formyl chloride with silver cyanate (See, Kim, D.K. et al., J. Heterocyclic Chem. 1995, 32, 1625). Alternatively, N-oxycarbonylcarbamate (or N-oxycarbonylurea) can be prepared in two steps. Step 1: reaction of an alcohol or phenol with chlorocarbonyl isocyanate to give N-oxycarbonyl carbamoyl chloride intermediate **(IP15B).** Step 2: reaction of the intermediate **IP15B** with the same or another alcohol or phenol or an amine. (For a review on chemistry of chlorocarbonyl isocyanate, see, Gorbatenko, V. I. Tetrahedron, 1993, 49, 3227).

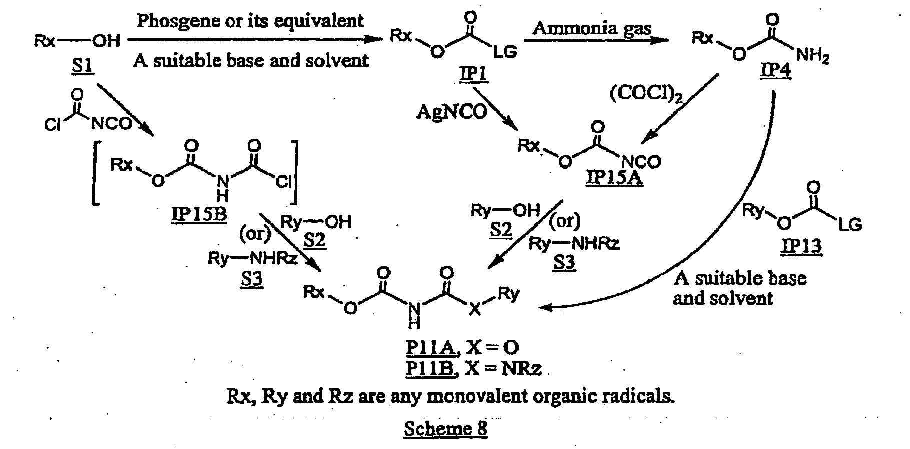

Suitable bases and solvents are same as defined above.

Compounds (Prodrugs) of the formula (I) containing bio-cleavable linkers and linkages can be synthesized by various methods obvious to those skilled in the art. As a matter of illustration, any of the approaches shown in the following schemes can be used to make such prodrugs of the formula (I) described herein.

Monoprotection of diol or aminoalcohol or diamino compounds [i.e., linker(s)] with suitable protecting groups and their selective removal at appropriate stage of the synthesis are carried out as described in Theodora W. Greene and Peter G.M. Wuts, "Protective Groups in Organic Synthesis", 3rd edition, John Wiley and Sons, Inc. New York (1999), the disclosures of which are incorporated herein by reference. Suitable protecting groups (PGs) include, acetyl, Boc, Fmoc, benzoyl, pivaloyl, trityl, tetrahydropyranyl (THP), and silyl (TBDMS, TMS, etc.). Obviously, selection of a suitable protecting group is very crucial for the success of a chosen method for the synthesis of prodrugs described in this invention.

Synthesis of appropriately derivatized/modified bio-labile linker is shown in Scheme 9.

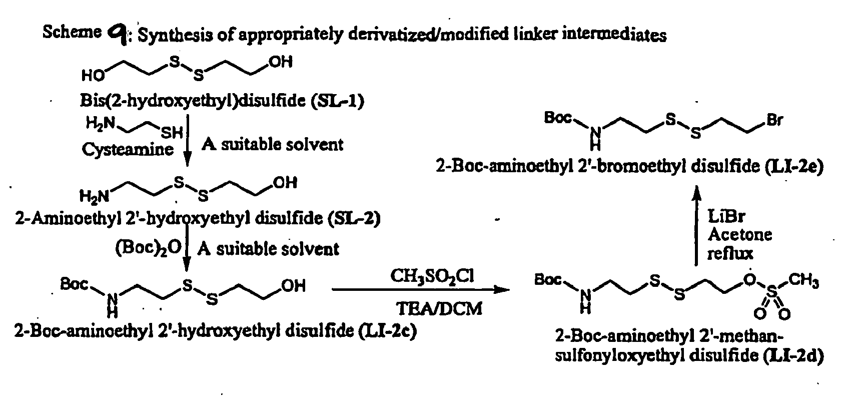

Double/Mutual prodrugs described in this invention can be synthesized by any of the approaches depicted in Schemes 10 through 12.

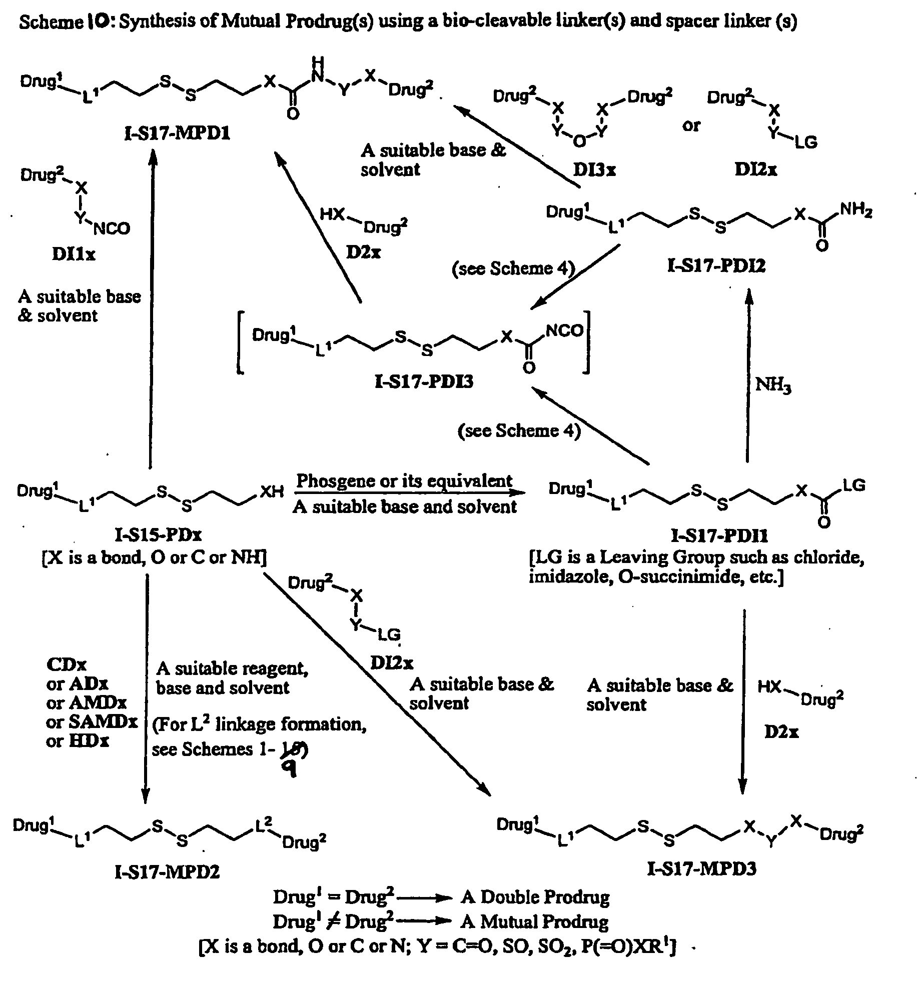

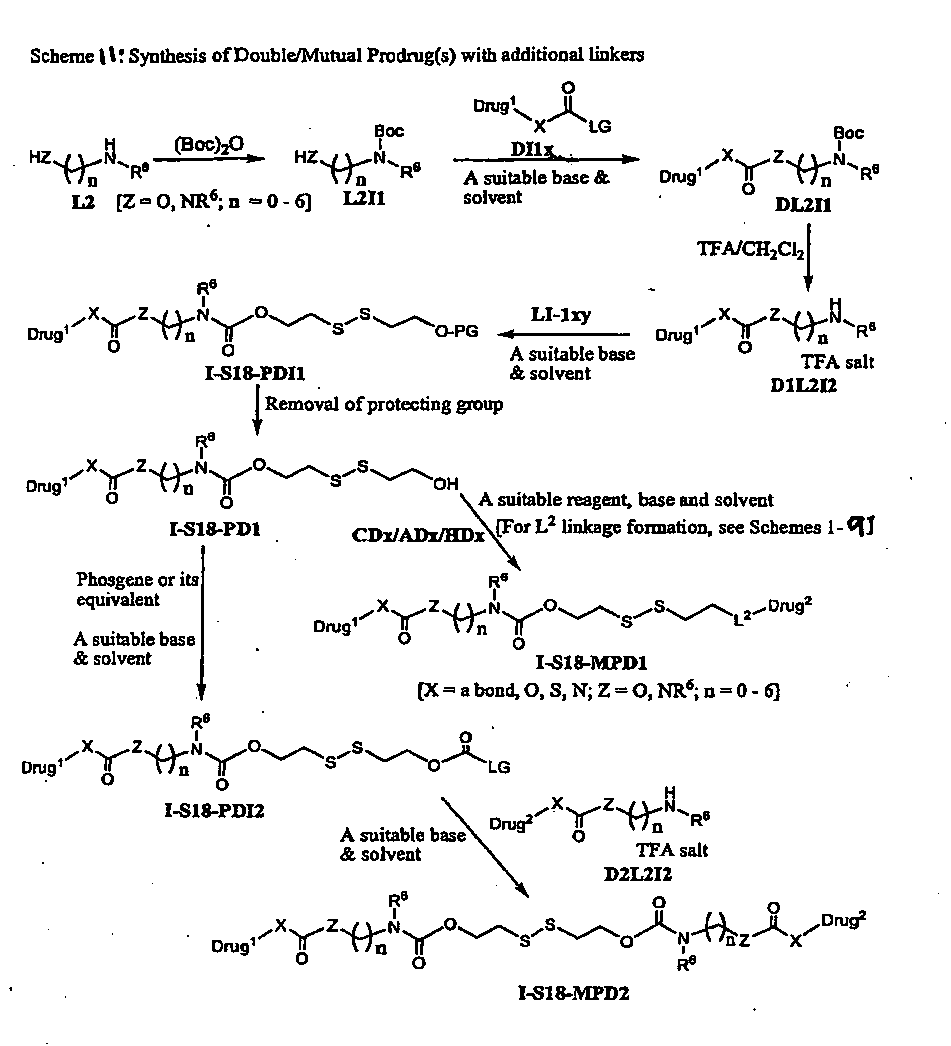

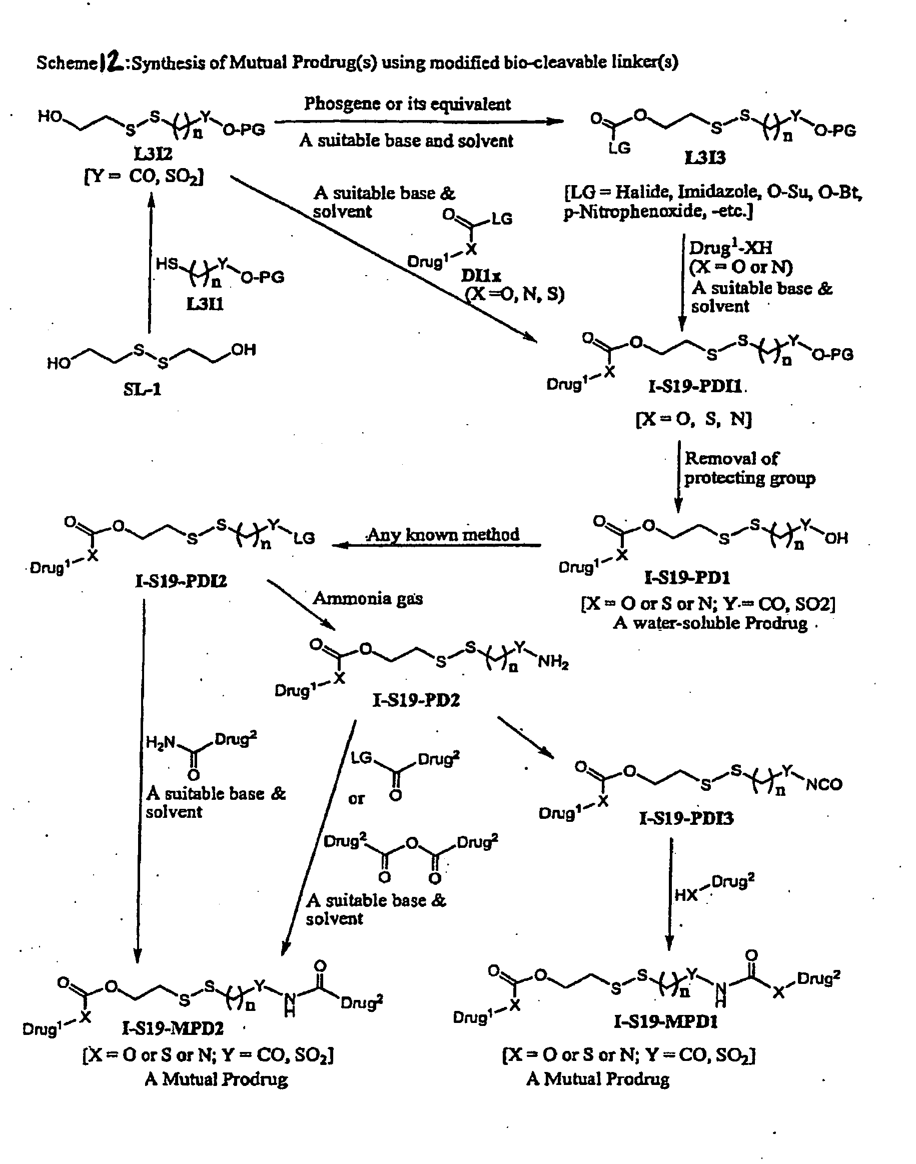

As a matter of illustration, mutual prodrug of desloratadine and pseudoephidrine was synthesized as depicted in Scheme 13.

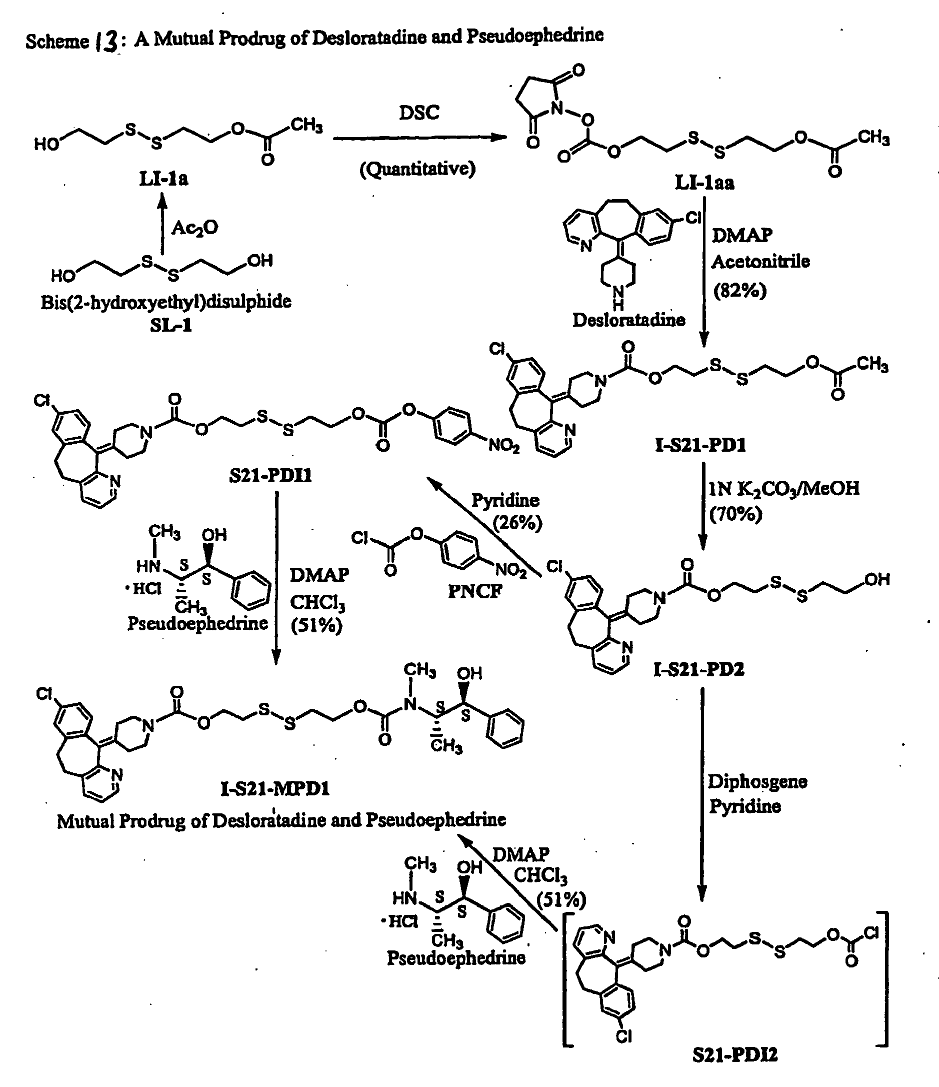

### Example 1

### Synthesis of 2-[(2-hydroxyethyl)dithio]ethyl acetate (LI-1a):

Acetic anhydride (5.67 ml, 56.87 mmol) and pyridine (40.4 ml, 499 mmol) were added to a solution of 2-(hydroxyethyl)disulfide **(SL-1,** 15.39 g, 99.78 mmol) in DCM (350 mL) at RT and the mixture was stirred at RT for 16 h. The mixture was concentrated and the residue, after usual aqueous work-up and chromatographic purification, afforded 8.16 g (42%) of **LI-1a** as a pale yellow oil. ¹H-NMR (300 MHz, CDCl₃): δ 2.00 (bs, 1H), 2.08(s, 3H), 2.80-2.95 (m, 4H), 3.89 (t, 2H, J = 6 Hz), 4.35 (t, 2H, J = 6 Hz), MS: (m/z) 219 [M]⁺.

### Example 2

### Synthesis of 2-{[2-(tetrahydro-2H-pyran-2-yloxy)ethyl]dithio}ethanol (LI-1b):

This compound was synthesized by a method described by K. F. Bernady et al., J. Org. Chem., 1979, 44, 1438. Dihydropyran (8.41 g, 100 mmol) was added to a solution of **SL-**1 (15.4 g, 100 mmol) in DCM (200 mL) at 0-5 °C, followed by PTSA (∼5%) and stirred at RT for 5 h. The mixture, after usual aqueous work-up and chromatographic purification, afforded 14.5 g (50%) of **LI-1b.** ¹H-NMR (300 MHz, CDCl₃): δ 1.5-1.9 (m, 6H), 2.88 (t, 2H, J = 6 Hz), 2.94 (t, 2H, J = 6 Hz), 3.45-3.57 (m, 1H), 3.67-3.78 (m,1H), 3.85-4.05 (m, 2H), 3.90 (t, 2H, J = 6 Hz), 4.65 (s, 1H).

### Example 3

### Synthesis of 2-{[2-(Trityloxy)ethyl]dithio}ethanol (LI-1c):

This compound was synthesized by a method described by O. Hernandez et al., Tetrahedron Letters, 1981, 22, 1491-1494. Thus, 8.58 g (21.4 mmol) of 4-dimethylamino-N-triphenylmethylpyridinium chloride (A.V. Bhatia et al., Organic Synthesis, 1997, 75, 184-185) was added to a solution of **SL-1** (3.0 g, 19.45 mmol) in DCM (90 mL) and stirred at RT for 24 h. The mixture, after usual aqueous work-up and chromatographic purification, afforded 2.86 g (37%) of **LI-1c.** ¹H-NMR (300 MHz, CDCl₃): δ 2.70 (t, 2H, J = 6.0 Hz), 2.88 (t, 2H, J = 6.0 Hz), 3.39 (t, 2H, J = 6.0 Hz), 3.80 (q, 2H, J = 6.0 Hz), 7.24-7.33 (m, 10H), 7.44-7.46 (m, 5H). MS (m/z): 396 [M]⁺.

### Example 4

### Synthesis of chloroacetic acid 2-(2-hydroxyethyldisulfanyl)ethyl ester (LI-1d):

To a solution of **SL-1** (23 g, 150 mmol) in DCM (250 mL) at 0 °C were added TEA (10.12 g, 100 mmol) and chloroacetyl chloride (11.3 g, 100 mmol) and stirred overnight at RT. The reaction mixture was concentrated and purified by column chromatography to afford 8.3 g (37%) of **LI-1d.** ¹H-NMR (300 MHz, CDCl₃): δ 2.88 (t, 2H, J = 5.7 Hz), 2.95 (t, 2H, J = 6.6 Hz), 3.89 (t, 2H, J = 5.7 Hz), 4.09 (s, 2H), 4.47 (t, 2H, J = 6.6 Hz).

### Example 5

Synthesis of tert-butyl 2-[(2-hydroxyethyl)dithio]ethylcarbamate **(LI-2c):** To a solution of cysteamine hydrochloride (15 g, 132 mmol) in MeOH (130 mL) at 0-5 °C was added TEA (37 mL, 264 mmol), followed by a solution of **SL-1** (20.4 g, 132 mmol) in DCM (50 mL) and stirred at RT for 6 h. The mixture, which contained the intermediate SL-2, was cooled and (Boc)₂O (63.4 g, 290.4 mmol) was added and stirred overnight. MeOH was removed under vacuum. After usual aqueous work-up and chromatographic purification, **LI-2c** was obtained as a colorless oil (14.6 g, 44%).

The above linker intermediate can also be prepared by the following method:
Step 1: TEA (37 ml, 264 mmol) and a solution of (Boc)₂O (48 g, 220 mmol) in DCM (100 mL) were added to a suspension of cystamine dihydrochloride (20 g, 88.8 mmol) in of DCM (300 mL) and stirred at RT for 15 h. The mixture was concentrated and the residue, after usual aqueous work-up and chromatographic purification, gave 30 g (96%) of tert-butyl 2-({2-[(tet-butoxycarbonyl)amino]ethyl}dithio)ethylcarbamate as a white solid. ¹H-NMR (300 MHz, CDCl₃): δ 1.43 (s, 18H), 2.78 (t, 4H, J = 6.3Hz), 3.44 (q, 4H, J = 6.0 Hz ), 5.00 (bs,1H). MS (m/z): 353.18 [M+H]⁺, 375.24 [M+Na]⁴⁺.
Step 2: A solution of 2-mercaptoethanol (1.44 g, 18.5 mmol) in DCM (10 mL) was added to a mixture of tert-butyl 2-({2-[(tert-butoxycarbonyl)amino]ethyl}dithio)ethyl carbamate (5.0 g, 14.2 mmol) and TEA (3.87 ml, 27.7 mmol) in DCM (30 mL) and stirred overnight at RT. After usual aqueous work-up and chromatographic purification, 2.0 g (56%) of **LI-2c** was obtained. ¹H-NMR (300MHz, CDCl₃): δ 1.43 (s, 9H ), 2.79 ( t, 2H, J = 6.5Hz ), 2.87 (t, 2H, J = 5.7Hz ), 3.48 (q, 2H, J = 6Hz ), 3.88 (t, 2H, J = 5.5 Hz), 4.8 (bs,1H). MS (m/z): 254 [M+H]⁺, 276.13 [M+Na]⁺.

Removal of the Boc group of **LI-2c** was accomplished as described in Example 10 to afford the TFA salt, **LI-2c.TFA.**

### Example 6

Synthesis of 2-Boc-aminoethyl-2'-methansulfonyloxyethyl disulfide **(LI-2d):** To an ice-cold solution of **LI-2c** (9 g, 35.52 mmol) in DCM (80 mL) and TEA (9.9 mL, 71.04 mmol) was added methanesulfonyl chloride (4.2 mL, 53.28 mmol). The reaction mixture was stirred at 0-5 °C for 45 min, then diluted with DCM. After usual aqueous work-up and chromatographic purification, 13.38 g of LI-2d were obtained, which was pure enough for further use. ¹H-NMR (300 MHz, CDCl₃): δ 1.43 (s, 9H), 2.80 (t, 2H, J = 6.4 Hz), 2.98 (t, 2H, 5.7 Hz), 3.05 (s, 3H), 3.35-3.45 (m, 2H), 4.45 (t, 2H, J = 6.7 Hz), 4.78 (br s, 1H).

### Example 7

Synthesis of 2-Boc-aminoethyl-2'-bromoethyl disulfide **(LI-2e):** To a solution of **LI-2d** (13 g, 39.27 mmol) in acetone (100 mL) at RT was added LiBr (6.82 g, 78.54 mmol) and stirred under reflux for 1 h. The reaction mixture was concentrated and the residue, after usual aqueous work-up and chromatographic purification, afforded 8.8 g (78%) of **LI-2e.** ¹H-NMR (300 MHz, CDCl₃): δ 1.44 (s, 9H), 2.80 (t, 2H, J = 6.32 Hz), 3.06 (t, 2H, J = 6.73 Hz), 3.44 (q, 2H), 3.61 (t, 2H, J = 7.62 Hz), 4.87 (br s, 1H). MS (EI)⁺ m/z: 317 [M+H]⁺.

### Example 8

Synthesis of methyl [(2-hydroxyethyl)dithio]acetate **(L3I2a):** Methyl mercaptoacetate (10.32 g, 97.4 mmol) was added to a solution of **SI-1** (10.0 g, 64.93 mmol) in DCM (150 mL) at RT, followed by TEA (18 mL, 129 mmol) and the mixture was stirred overnight at RT. After usual aqueous work-up and chromatographic purification, 2.7 g (22.9 %) of **L3I2a** were obtained. ¹H-NMR (300 MHz, CDCl₃): δ 2.95 (t, 2H, J = 2.5 Hz), 3.49 (s, 2H), 3.76 (s, 3H), 3.86 (q, 2H, J = 5.64). MS (m/z): 182 [M+H]⁺.

### Example 9

Synthesis of prodrug **I-C1-PD10:** This prodrug was synthesized as described in Scheme 11, Method B. Thus, TEA (0.73 mL, 1.0 mmol) was added to a suspension of cetirizine dihydrochloride (2.0 g, 4.68 mmol) in DCM (50 mL), followed by a solution of **SL-1** (0.72 g, 4.67 mmol), DCC (1.13 g, 5.47 mmol) and DMAP (0.112g, 1 mmol) and stirred at RT for 15 h. The mixture was concentrated and the residue, after usual aqueous work-up and chromatographic purification, gave 0.44 g (19%) of **I-C1-PD10.** ¹H-NMR (300 MHz, CDCl₃): δ 2.50 (bs, 4H), 2.80 (bs, 6H), 2.87 (t, 2H, J = 6.09 Hz), 2.94 (t, 2H, J = 7.32 Hz), 3.75 (m, 2H), 3.86 (t, 2H, J = 6.12 Hz), 4.13 (s, 2H), 4.24 (s, 1H), 4.40 (t, 2H, J = 6.09 Hz) and 7.22-7.35 (m, 9H). MS (m/z): 527 [M+H]⁺.

The following double/mutual prodrugs (Examples 10 and 11B) were synthesized by the methods depicted in Schemes 10 to 13, using appropriate therapeutic agents and obvious modifications:

### Example 10

Synthesis of mutual prodrug of desloratidine and pseudoephedrine **(I-AA-MPD1):**
This mutual prodrug was synthesized as depicted in Scheme 13. The compound **I-AA-MPD1** was obtained as a colorless gum. ¹H-NMR (300 MHz, CDCl₃): δ 1.00 (d, 3H, J = 6.9 Hz), 2.27-2.51 (m, 4H), 2.74-2.97 (m, 9H), 3.28-3.41(m, 4H), 3.79 (bs, 2H), 4.28-4.30 (m, 4H), 4.57 (m, 1H), 7.04-7.44 (m, 9H), 8.26-8.33 (m, 2H). MS (m/z): 682 [M+H]⁺.

### Example 11A [Not according to the claimed invention]

### Synthesis of mutual prodrug of zidovudine and lamivudine (I-HH-MPD1):

### Step 1: Synthesis of intermediate I-S17-PDI1:

4-Nitrophenyl chloroformate (0.27 g, 1.34 mmol) was added to a solution of the **I-H1-PDS** (0.4 g, 0.89 mmol) and pyridine (76 µL, 1 mmol) in DCM (10 mL) and stirred at RT for 15 h. The mixture was concentrated and the residue purified by column chromatography to give 0.29 g (53%) of **I-S17-PDI1.** ¹H-NMR (300 MHz, CDCl₃): δ 1.93 (s, 3H), 2.45 (m, 2H), 2.97-3.06 (m, 4H), 4.05 (m, 1H), 4.41 (m, 1H), 4.40-4.49 (m, 4H), 4,54 (t, 2H, J = 6.5 Hz), 6.17 (t, 1H, J = 6.0 Hz), 7.33 (s, 1H), 7.39 (d, 2H, J= 4.8 Hz), 8.28 (d, 2H, J = 4.8 Hz) and 8.50 (s, 1H). MS (m/z): 635 [M+Na]⁺.

### Step 2: Synthesis of I-HH-MPD1:

Lamivudine (45 mg, 0.196 mmol) and DMAP (48 mg, 0.39 mmol) were added to a solution of **I-S17-PDI1** (80 mg, 0.13 mmol) in DMF (1.5 mL) and stirred at RT for 30 min. The mixture was concentrated and purified by column chromatography to give 40 mg (43%) of product **1-HH-MPD1.** ¹H-NMR (300 MHz, CDCl₃): δ 1.90 (s, 3H), 2.45 (t, 2H, J = 6.1 Hz), 3.05 (t, 4H, J = 6.2 Hz), 3.20 (m, 1H), 3.53 (m, 1H), 4.08 (m, 1H), 4.30-4.80 (m, 8H), 5.45 (t, 1H, J = 3.0 Hz), 5.90 (d, 1H, J = 7.5 Hz), 6.17 (t, 1H), 6.30 (t, 1H), 7.55 (s, 1H) and 7.90 (d, 1H, J = 7.50 Hz). MS (m/z): 725 [M+Na]⁺.

### Example 11B

### Synthesis of mutual prodrug of cetirizine and pseudoephedrine (I-CA-MPD1):

### Step 1: Synthesis of intermediate I-S17-PDI1:

This intermediate was prepared by reacting **I-C1-PD10** with p-nitrophenyl chloroformate by a procedure similar to that described in Example 11A. The desired intermediate **I-S17-PDI1** was obtained as a gum. ¹H-NMR (300 MHz, CDCl₃): δ 2.49-2.71 (m, 10H), 2.95 (t, 2H, J = 6.6Hz), 3.01 (t, 2H, J = 6.5 Hz), 3.73 (bs, 2H), 4.13 (s, 2H), 4.22 (s, 1H), 4.41 (t, 2H, J = 6.6 Hz), 4.53 (t, 2H, J = 6.6 Hz), 7.18-7.40 (m, 11 H), 8.28 (d, 2H, J = 7.1 Hz).
Step 2: The mutual prodrug **I-CA-MPD1** was synthesized by reacting intermediate **I-S17-PDI1** with pseudoephidrine by a procedure similar to that described in Example 11A, Step 2. The desired mutual prodrug **I-CA-MPD1** was obtained as a colorless gummy material. ¹H-NMR (300 MHz, CDCl₃): δ 0.99-1.09 (d, 3H, J = 6.6 Hz), 2.45 (bs, 4H), 2.68 (bs, 6H), 2.90 (s, 3H), 2.91-2.94 (m, 4H), 3.71 (bs, 3H), 4.11 (s, 2H), 4.18 (s, 1H), 4.26-4.41 (m, 4H), 4.56 (m, 2H), 7.17- 7.35 (m, 12H). MS (m/z): 716 [M+ H]⁺.

## Claims

1. A compound of formula (I) or pharmaceutically acceptable salts thereof: 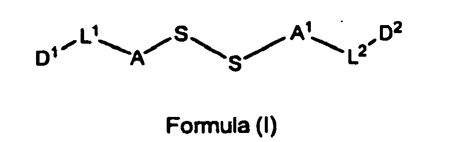 wherein,
A and A¹ independently represent a bond, (CH₂)_{d}, 1,2-phenylene, 1,3-phenylene or 1,4-phenylene;
d is 1-8;
D¹ and D² are independently selected from: cetirizine, desloratidine, terfenadine, fexofenadine and pseudoephedrine;
L¹ and L² independently represent a bond, O, S, NR¹, L, or a linkage selected from the group consisting of: 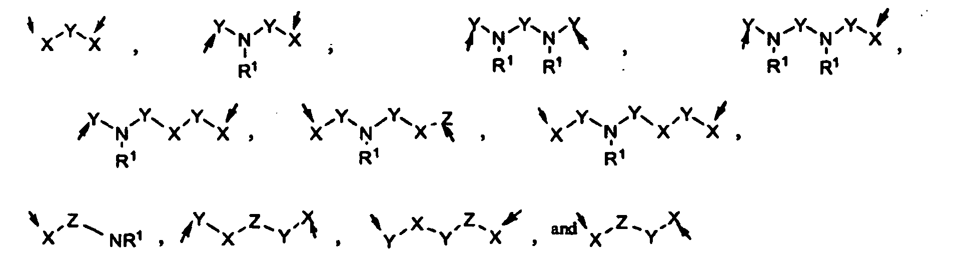
L is a group with bonding in any direction, independently selected from the group consisting of: 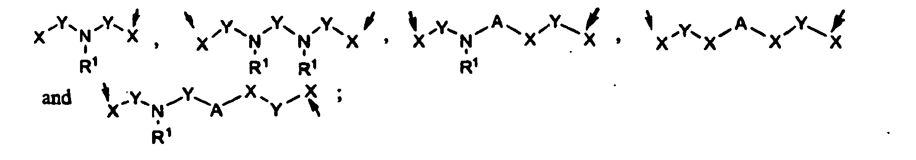 where
X independently represents a bond, C, O, S, or NR¹;
Y independently represents a bond, C=O, C=S, S=O, SO₂, P(=O)XR¹, or (CH₂)_{d}; wherein d is as defined;
Z independently represents a bond, or (CH₂); wherein, j is 1-4;
R¹ independently represents a bond, H, (C₁-C₈)alkyl, substituted(C₁-C₈)alkyl, (C₅-C₁₄)aryl, aralkyl or M^{e+};
M independently represents Na, K or a pharmaceutically acceptable metal ion; and
e = 1-3.

2. The compound according to claim 1 wherein, L² is O; A and A¹ are independently (CH₂)_{d}, 1,2-phenylene, 1,3-phenylene, or 1,4-phenylene; and d is 1-8.

3. The compound according to claim 1, wherein A and A¹ are (CH₂)₂ and D² is the same as D¹.

4. The compound according to claim 1, wherein D² is the same as D¹.

5. The compound according to claim 3, wherein the compound is selected from: 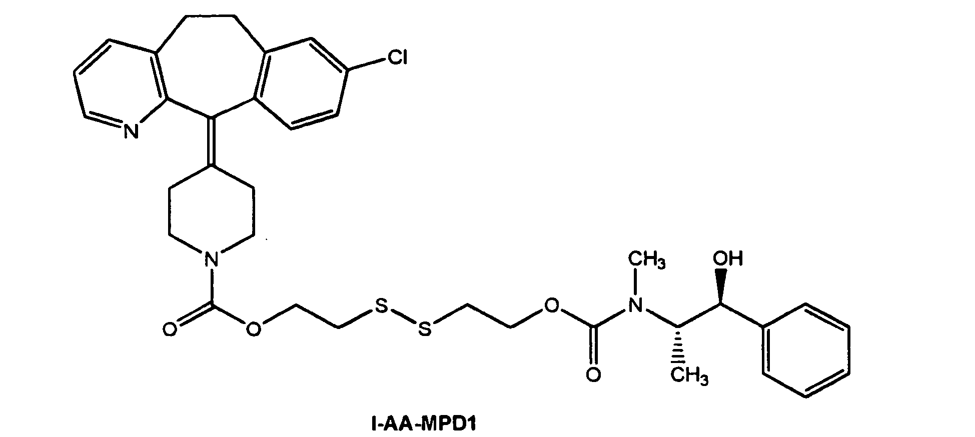 and

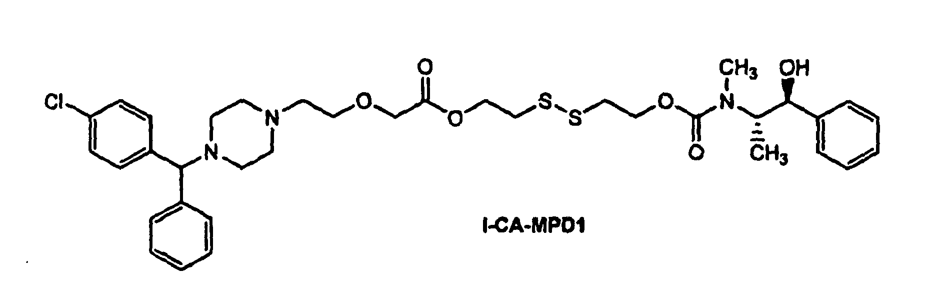

6. A compound as claimed in claim 1 wherein the compound is selected from I-CA-MPD2, I-CA-MPD3, I-CA-MPD4 and I-AH-MPD16 or pharmaceutically acceptable salts thereof.

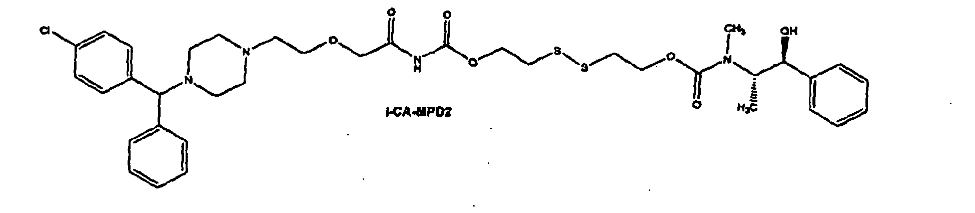

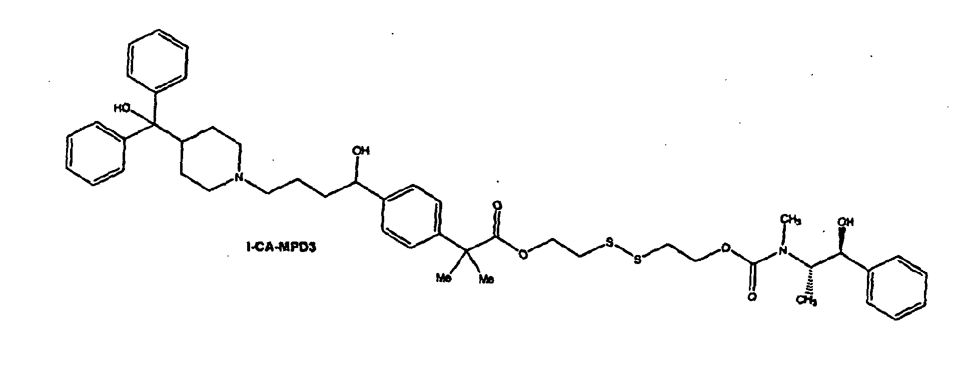

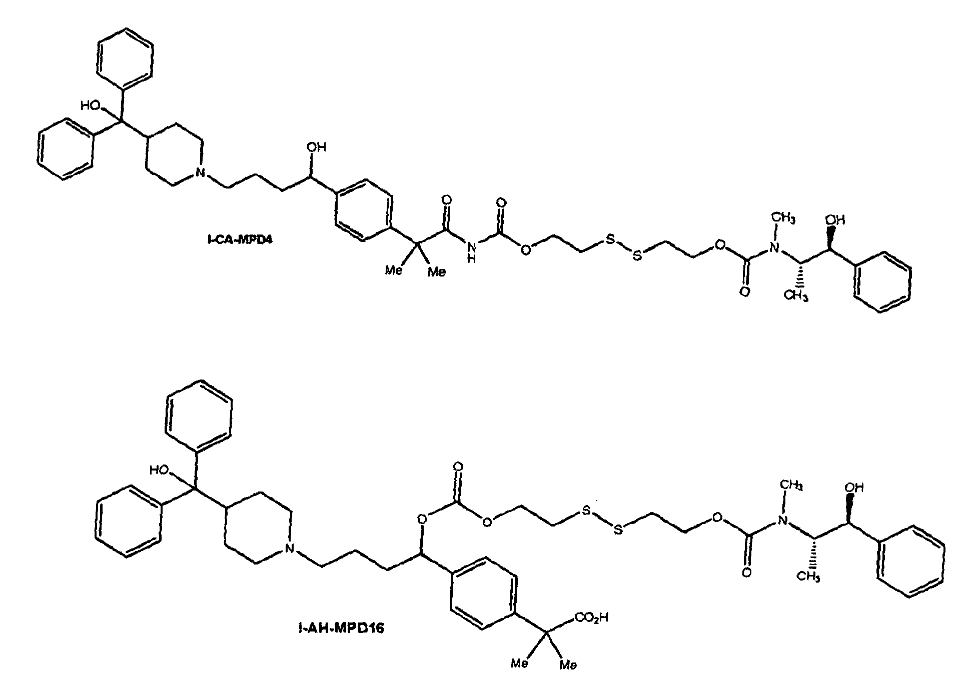

7. A pharmaceutical composition comprising a therapeutically effective amount of the compound as defined in any one of the claims 1 to 6, or a pharmaceutical salt thereof and one or more pharmaceutically acceptable carriers, vehicles or diluents.

8. A compound of claim 1, wherein D¹ and D² as pairs of drugs are selected from: Pseudoephedrine and Fexofenadine/Cetirizine/Desloratadine.

9. A compound as defined in any one of the claims 1 to 6 and 8, for use in the treatment of diseases where a chronic, sustained and selective release of the constituent drug is beneficial.

10. A composition as claimed in claim 7, for use in the treatment of diseases where a chronic, sustained and selective release of the constituent drug(s) is beneficial.

11. The intermediates obtained in the preparation of compounds of claim 1, wherein the intermediates are selected from:

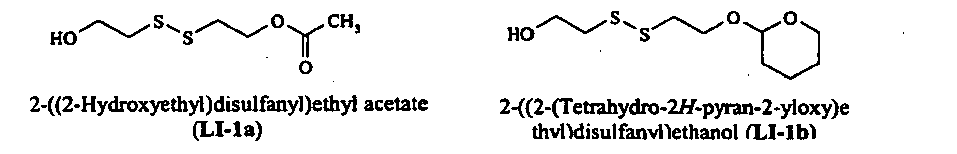

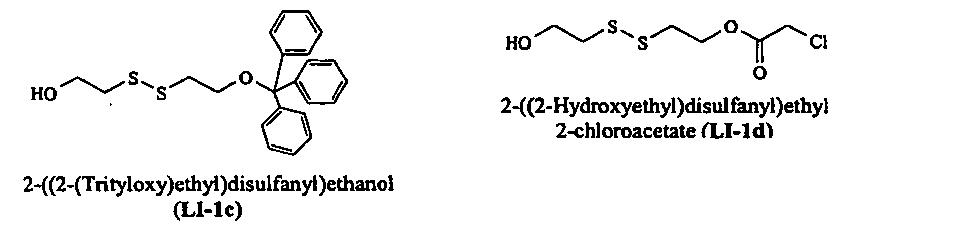

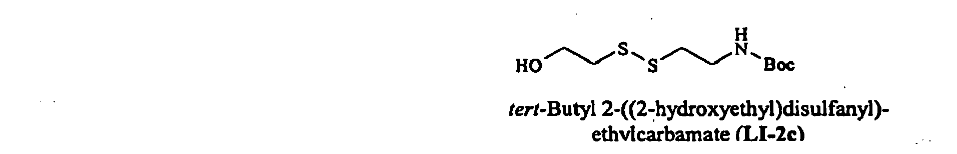

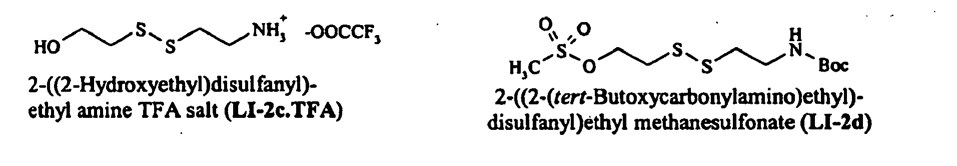

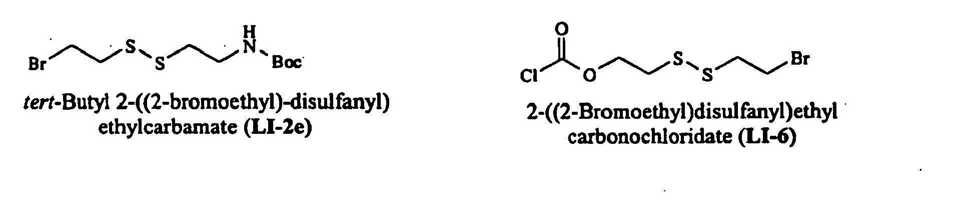

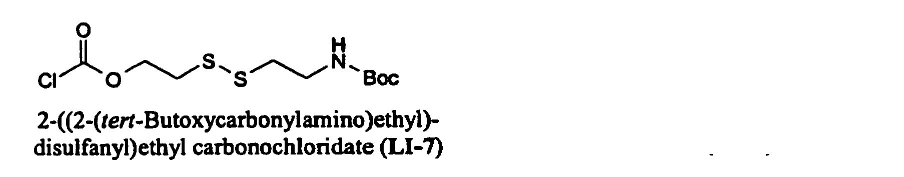

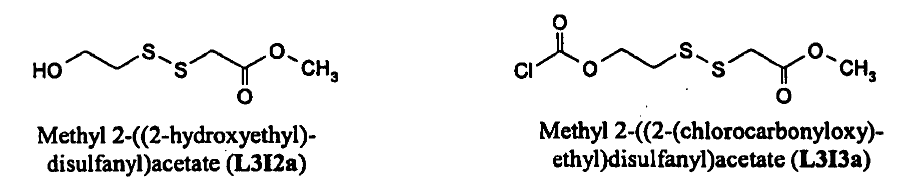

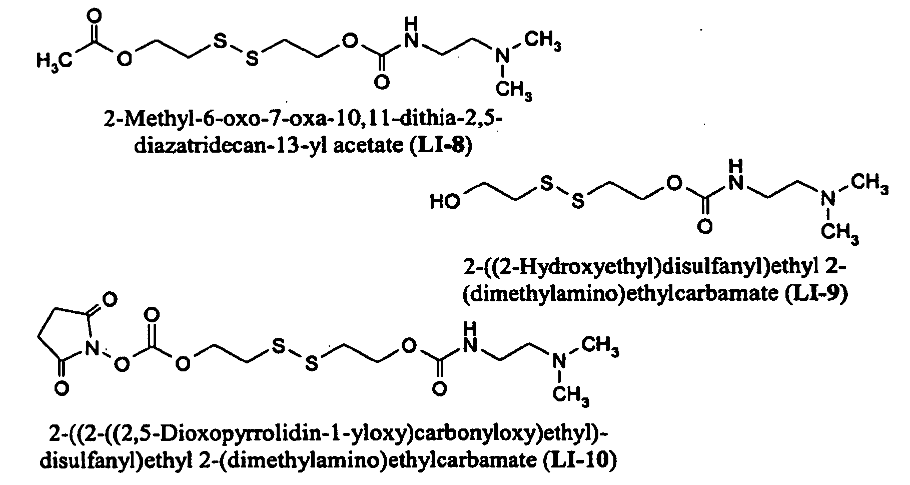

12. A process for the preparation of the compounds as claimed in claim 1, wherein the preparation of said compounds involves use of the intermediates selected from a group consisting of:

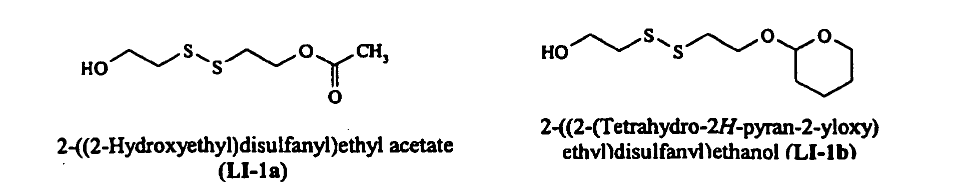

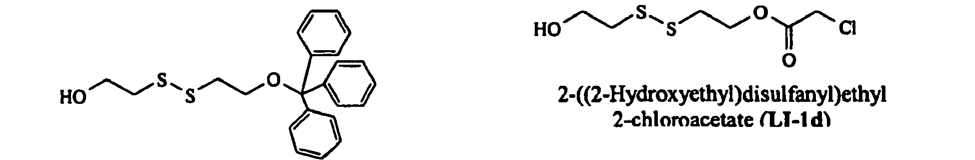

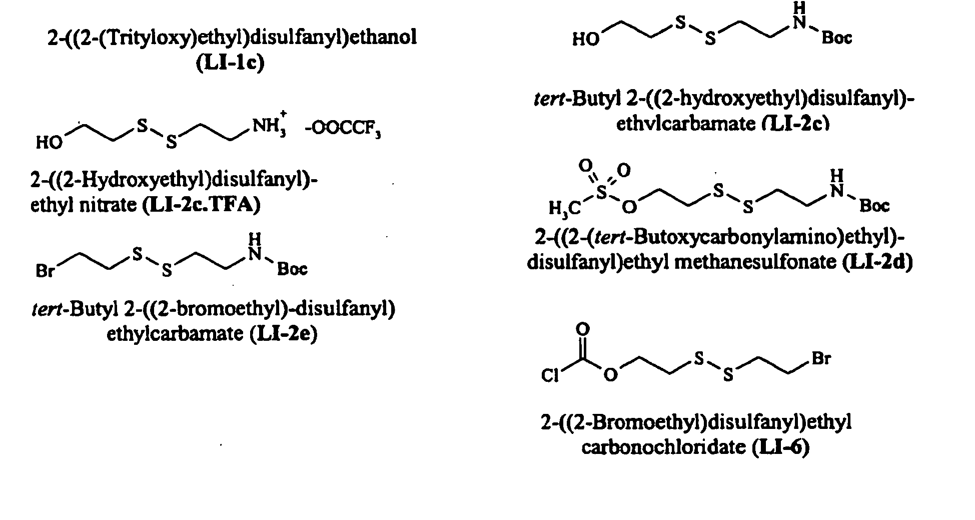

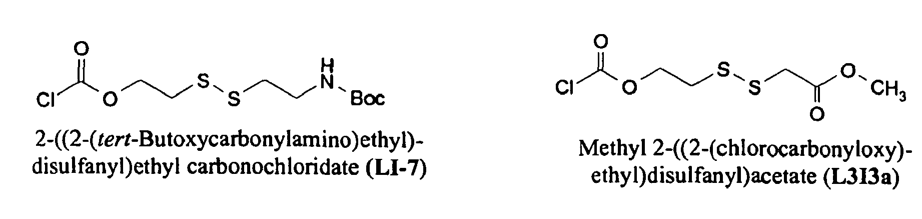

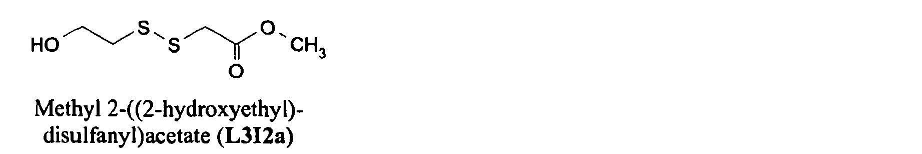

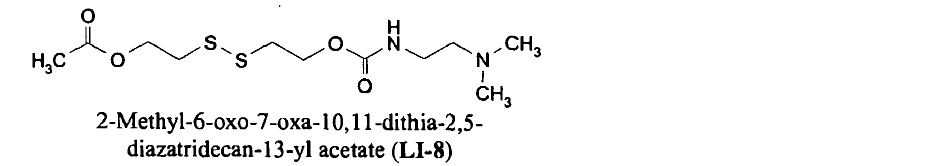

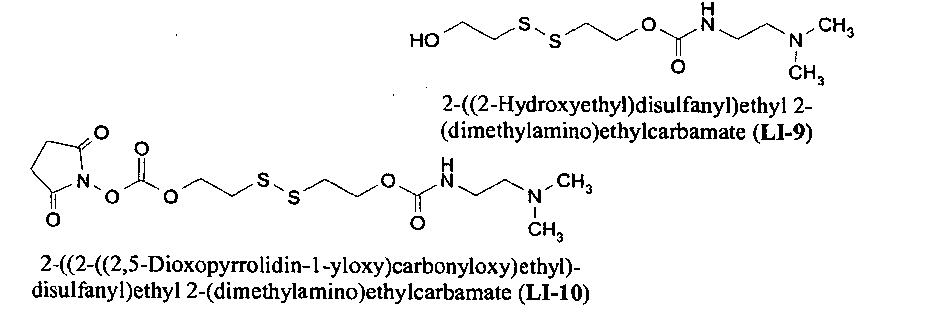

13. A process for the preparation of the compound as in claim 1, or a pharmaceutically acceptable salts thereof, wherein the process is selected from:
Process 1:
A ) Monoprotection of Bis-(2-hydroxyethyl)disulphide (SL-1) with an appropriate hydroxyl protecting group to give the corresponding monoprotected intermediate LI-1x 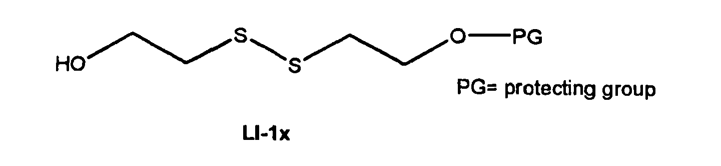
B) Converting the monoprotected intermediate LI-1x obtained in step A to an activated formyl intermediate LI-1 xy by treating with phosgene or its equivalent,
and
C) Reacting the activated formyl intermediate LI-1xy obtained in the step B with an appropriate amino- or hydroxyl containing drug (D¹) to give the corresponding compound of formula I;
Process 2:
A) Converting carboxyl containing drug (D¹) into its activated acyl halide or imidazolide or isocyanate by known methods, and
B) Reacting the intermediate obtained in the step A with the monoprotected linker intermediate LI-1x to obtain the compound of formula I;
Process 3:
A) Monoprotection of Bis-(2-hydroxyethyl)disulphide (SL-1) with an appropriate hydroxyl protecting group to give the corresponding monoprotected intermediate LI-1x,
B) Reacting formyl linker intermediate LI-1xy with amino or hydroxyl containing drug (D¹) to obtain the prodrug of formula I with free hydroxyl group on the linker,
C) Converting the intermediate obtained in the step B into activated formyl halide or imidazolide derivative, and
D) Reacting the intermediate obtained in the step C with the drug D² to obtain the mutual prodrug of formula I.

## Patentansprüche

1. Eine Verbindung der Formel (I) oder pharmazeutisch geeignete Salze davon: 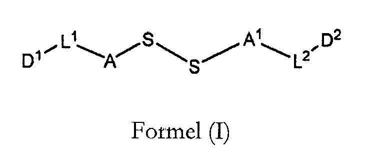
wobei A und A¹ unabhängig voneinander eine Bindung, (CH₂)_{d}, 1,2-Phenylen, 1,3-Phenylen oder 1,4-Phenylen darstellen;
d 1-8 ist;
D¹ und D² unabhängig voneinander ausgewählt sind aus: Cetrizin, Desloratidin, Terfenadin, Fexofenadin und Pseudoephedrin;
L¹ und L² unabhängig voneinander eine Bindung, O, S, NR¹, L darstellen oder ein Verbindungsstück, ausgewählt aus der Gruppe bestehend aus: 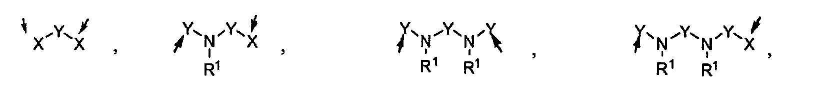 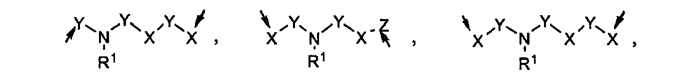 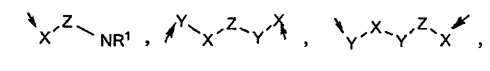 und 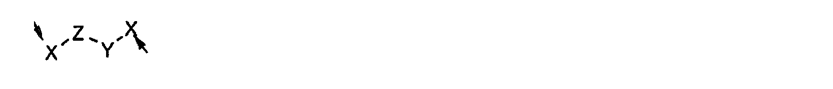
L eine Gruppe mit einer Bindung in einer beliebigen Richtung ist, unabhängig ausgewählt aus der Gruppe bestehend aus: 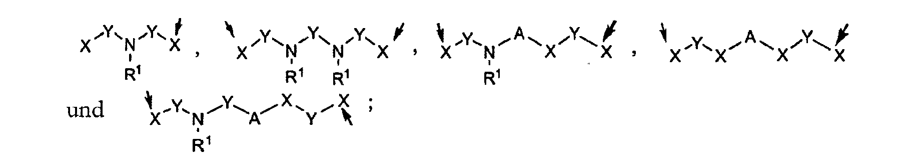 wobei
X unabhängig eine Bindung, C, O, S, oder NR¹ darstellt;
Y unabhängig eine Bindung, C=O, C=S, S=O, SO₂, P(=O)XR¹, oder (CH₂)_{d} darstellt; wobei d wie definiert vorliegt;
Z unabhängig eine Bindung oder (CH₂)ⱼ darstellt; wobei j 1-4 ist;
R¹ unabhängig eine Bindung, H, (C₁-C₈) Alkyl, substituiertes (C₁-C₈) Alkyl, (C₅-C₁₄) Aryl, Aralkyl oder M^{e+} darstellt;
M unabhängig Na, K oder ein pharmezeutisch geeignetes Metallion darstellt, und
e= 1-3.

2. Die Verbindung gemäß Anspruch 1, wobei L² O ist; A und A¹ unabhängig voneinander (CH₂)_{d}, 1,2-Phenylen, 1,3-Phenylen, oder 1,4-Phenylen sind; und d 1-8 ist.

3. Die Verbindung gemäß Anspruch 1, wobei A und A¹ (CH₂)₂ sind und D² das Gleiche wie D¹ ist.

4. Die Verbindung gemäß Anspruch 1, wobei D² das Gleiche wie D¹ ist.

5. Die Verbindung gemäß Anspruch 3, wobei die Verbindung ausgewählt ist aus: 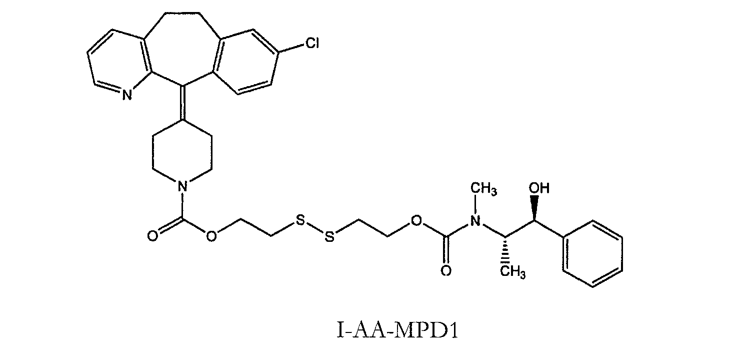 und

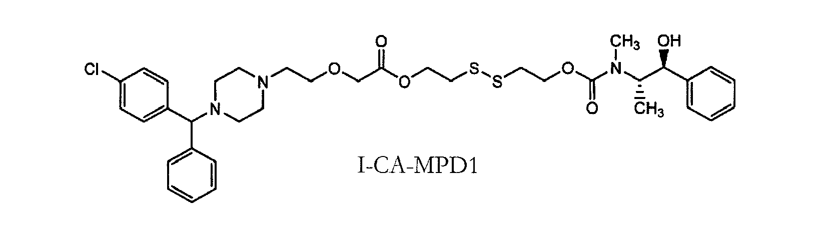

6. Eine Verbindung wie in Anspruch 1 beansprucht, wobei die Verbindung ausgewählt ist aus I-CA-MPD2, I-CA-MPD3, I-CA-MPD4 und I-AH-MPD16 oder pharmazeutisch geeigneten Salzen davon

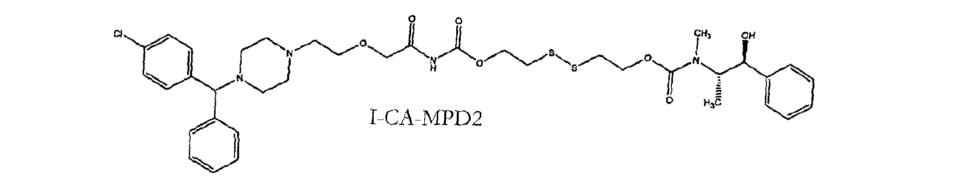

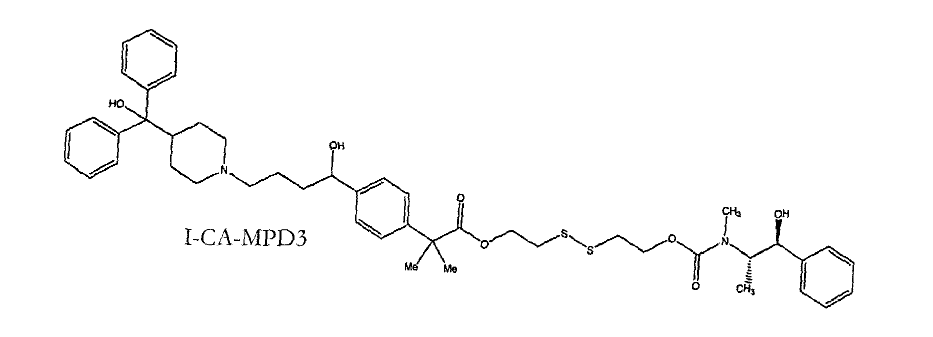

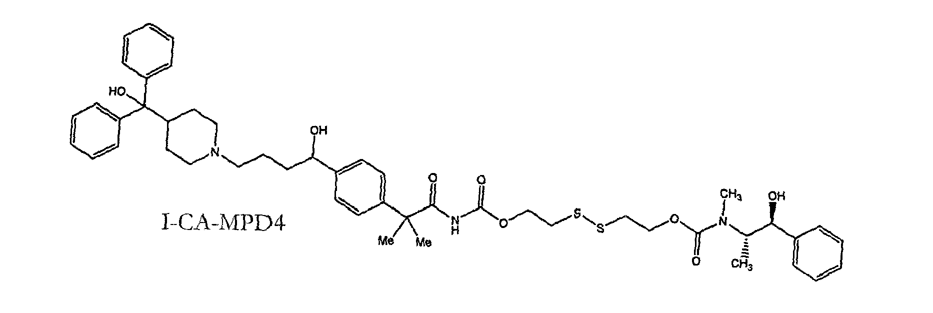

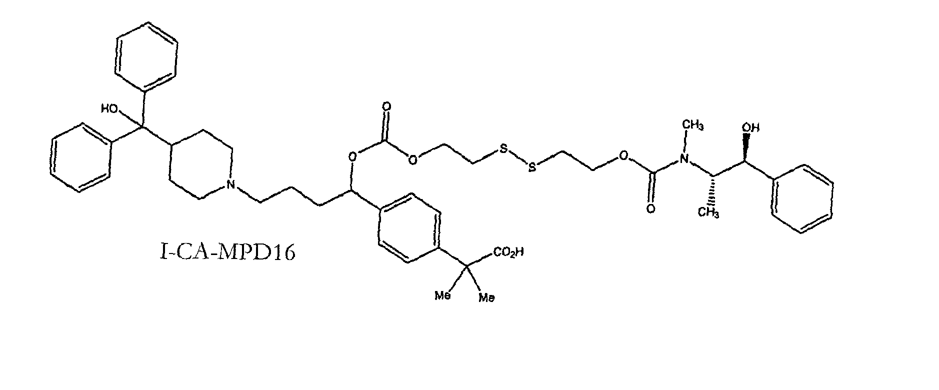

7. Eine pharmazeutische Zusammensetzung umfassend eine therapeutisch wirksame Menge der Verbindung wie in einem der Ansprüche 1 bis 6 definiert, oder ein pharmazeutisches Salz davon und einen oder mehrere pharmazeutisch geeignete Träger, Vehikel oder Verdünnungsmittel.

8. Eine Verbindung von Anspruch 1, wobei D¹ und D² als Paar von Medikamenten ausgewählt sind aus: Pseudoephidrin und Fexofenadin/Cetirizin/Desloratadin.

9. Eine Verbindung wie in einem der Ansprüche 1 bis 6 und 8 definiert, für die Verwendung in der Behandlung von Krankheiten, bei denen eine chronische, fortgesetzte und selektive Freisetzung des Medikamentenbestandteils vorteilhaft ist.

10. Eine Zusammensetzung wie in Anspruch 7 beansprucht, für die Verwendung in der Behandlung von Krankheiten, bei denen eine chronische, fortgesetzte und selektive Freisetzung des Medikamentenbestandteils oder der Medikamentenbestandteile vorteilhaft ist.

11. Die bei der Herstellung der Verbindungen von Anspruch 1 erhaltenen Zwischenprodukte, wobei die Zwischenprodukte ausgewählt sind aus:

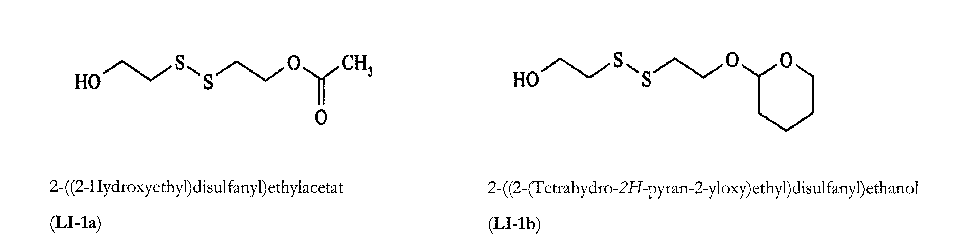

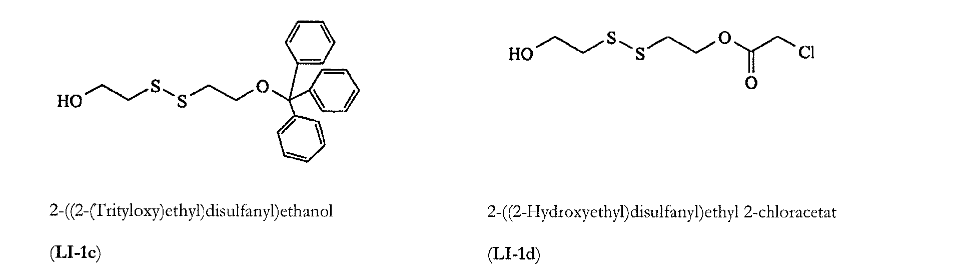

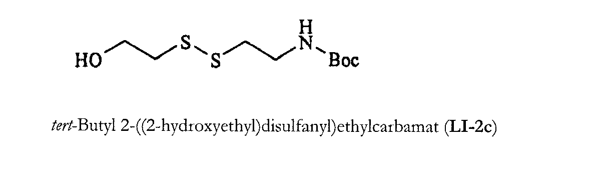

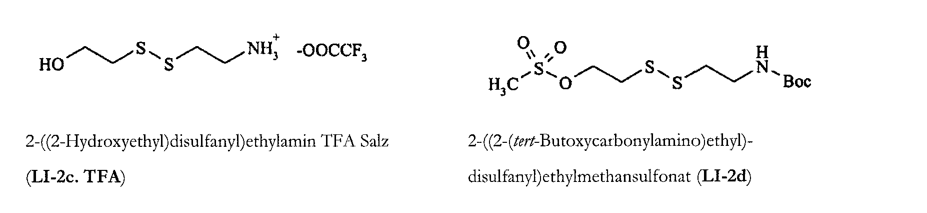

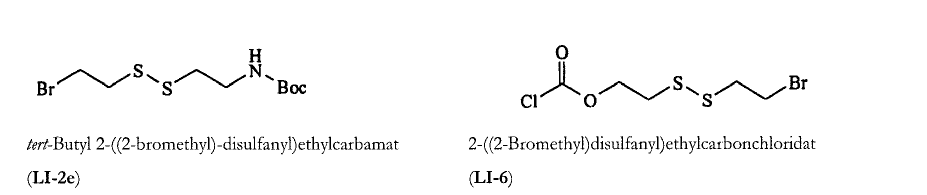

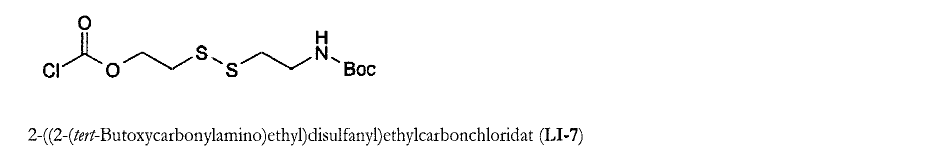

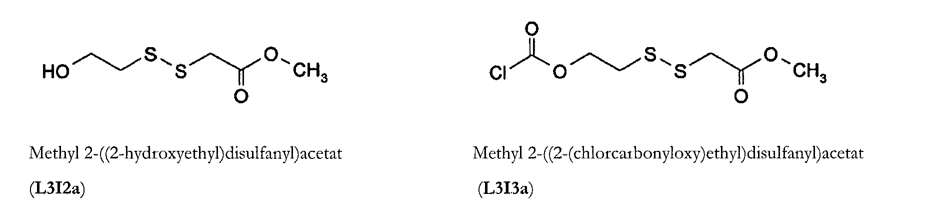

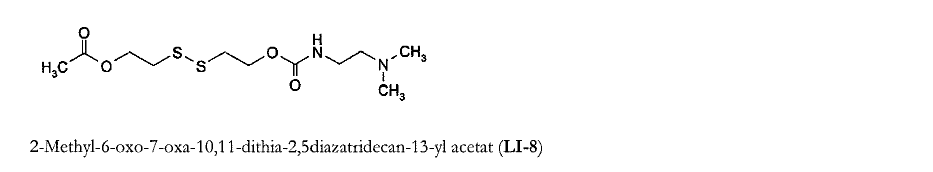

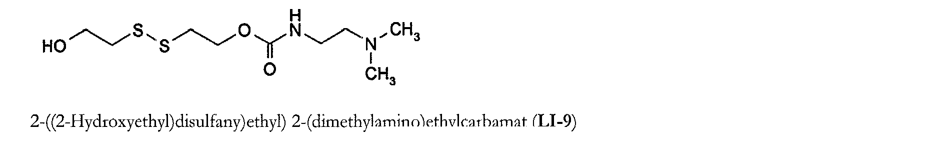

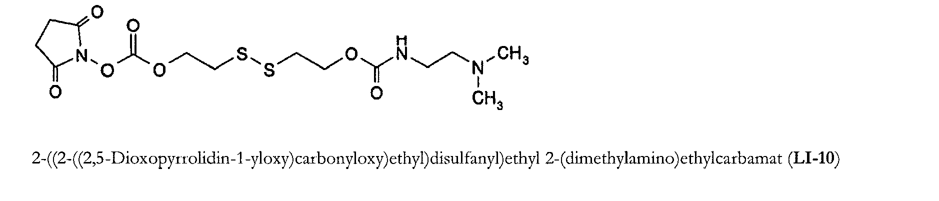

12. Ein Verfahren zur Herstellung der Verbindungen wie in Anspruch 1 beansprucht, wobei die Herstellung der besagten Verbindungen die Verwendung der Zwischenprodukte beinhaltet, die ausgewählt sind aus einer Gruppe bestehend aus:

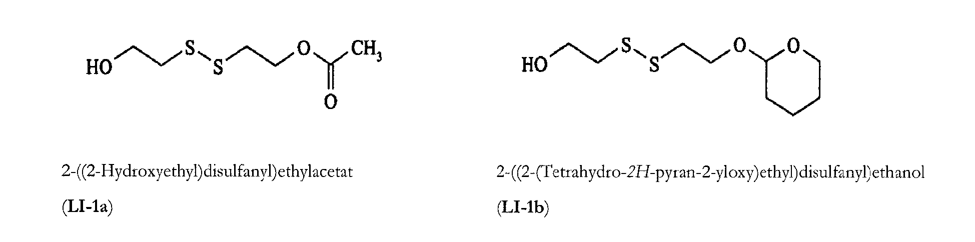

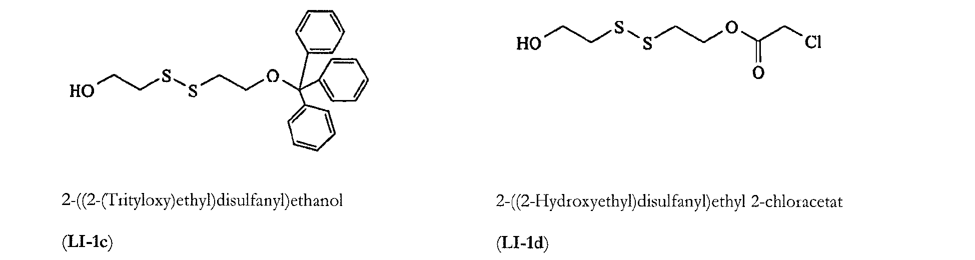

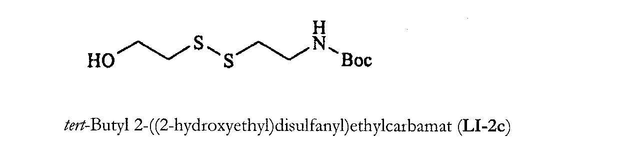

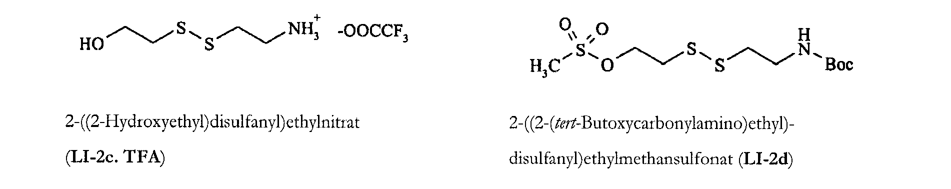

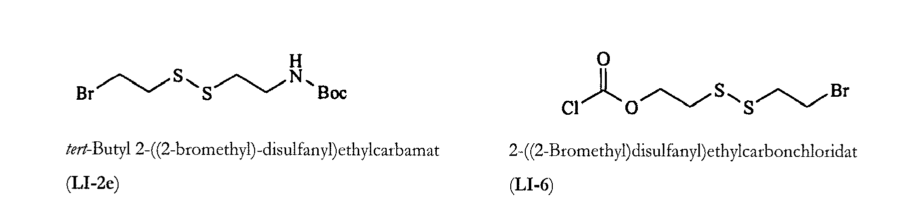

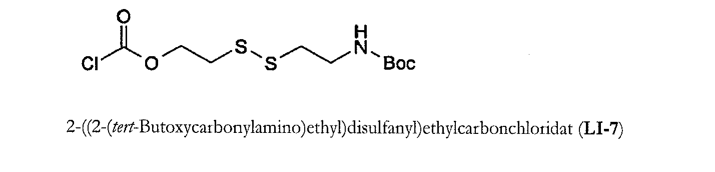

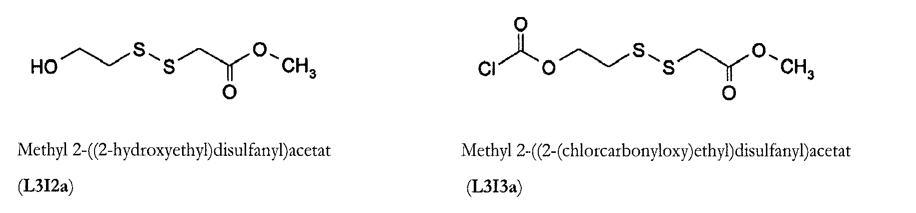

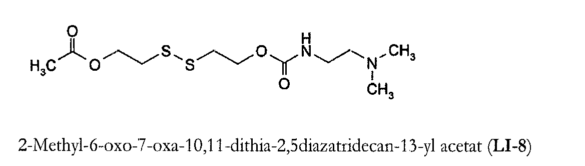

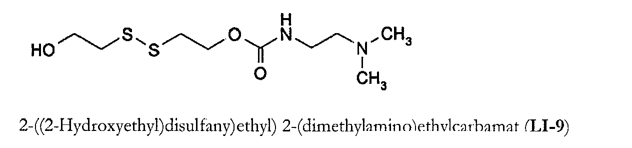

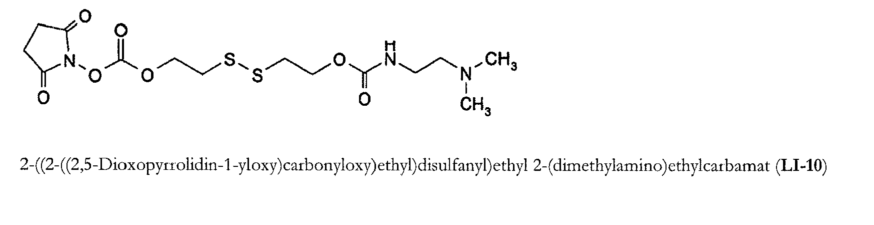

13. Ein Verfahren für die Herstellung der Verbindung wie in Anspruch 1, oder pharmazeutisch geeigneter Salze davon, wobei das Verfahren ausgewählt ist aus:
Verfahren 1:
A) Monoprotektion von Bis-(2-Hydroxyethyl)disufid (SL-1) mit einer geeigneten Hydroxyl-Schutzgruppe, um das zugehörige monoprotektierte Zwischenprodukt LI-1x zu erhalten 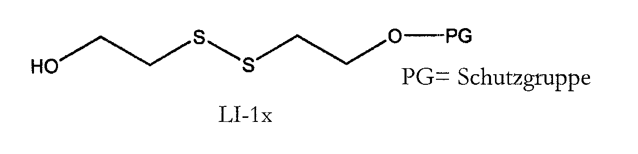
B) Umwandlung des in Schritt A erhaltenen monoprotektierten Zwischenprodukts LI-1x zu einem aktivierten Formyl-Zwischenprodukt LI-1xy durch Behandlung mit Phosgen oder dessen Äquivalent,
und
C) Zur Reaktion bringen des in Schritt B erhaltenen aktivierten Formyl-Zwischenprodukts LI-1xy mit einem geeigneten Amino- oder Hydroxyl-haltigen Medikament (D¹), so dass sich die zugehörige Verbindung der Formel I ergibt;
Verfahren 2:
A) Umwandeln des Carboxyl-haltigen Medikaments (D¹) in sein aktiviertes Acyl-Halid oder Imidazolid oder Isocyanat mittels bekannter Methoden, und
B) Zur Reaktion bringen des in Schritt A erhaltenen Zwischenprodukts mit dem monoprotektierten Linker-Zwischenprodukt LI-1x, um die Verbindung der Formel I zu erhalten;
Verfahren 3:
A) Monoprotektion von Bis-(2-Hydroxyethyl)disufid (SL-1) mit einer geeigneten Hydroxyl-Schutzgruppe, um das zugehörige monoprotektierte Zwischenprodukt LI-1x zu erhalten,
B) Zur Reaktion bringen des Formyl-Linker Zwischenprodukts L1-1xy mit dem Amino- oder Hydroxyl-haltigen Medikament (D¹), um die Medikamentenvorstufe der Formel I mit freier Hydroxylgruppe am Linker zu erhalten,
C) Umwandeln des in Schritt B erhaltenen Zwischenprodukts in aktiviertes Formylhalid- oder Imidazol-Derivat, und
D) Zur Reaktion bringen des in Schritt C erhaltenen Zwischenprodukts mit dem Medikament D², um die gemeinsame Medikamentenvorstufe der Formel I zu erhalten

## Revendications

1. Composé de formule (I) ou sels pharmaceutiquement acceptables de celui-ci : 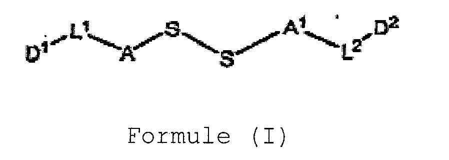 dans laquelle :
- A et A¹ représentent indépendamment une liaison, (CH₂)_{d}, 1,2-phénylène, 1,3-phénylène ou 1,4-phénylène ;
- d est 1-8 ;
- D¹ et D² sont indépendamment choisis parmi : la cétirizine, la desloratidine, la terfénadine, la fexofénadine et la pseudoéphédrine ;
- L¹ et L² représentent indépendamment une liaison, 0, S, NR¹, L ou une liaison choisie dans le groupe consistant en : 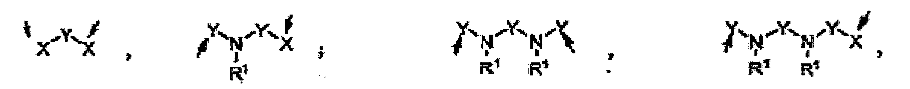 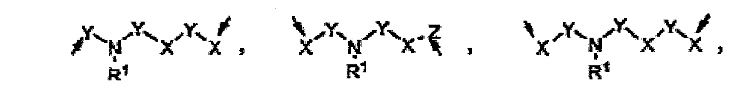 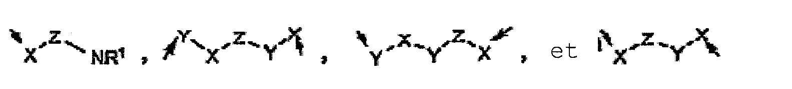
- L est un groupe avec une liaison dans n'importe quelle direction, choisi indépendamment dans le groupe consistant en : 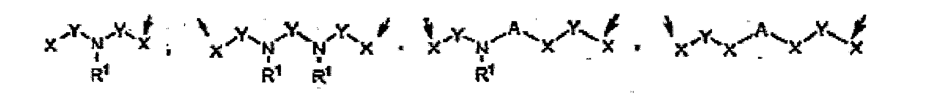 et 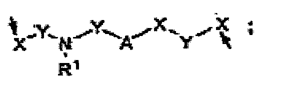 où
- X représente indépendamment une liaison, C, 0, S ou NR¹ ;
- Y représente indépendamment une liaison, C=O, C=S, S=O, SO₂, P(=O)XR¹ ou (CH₂)_{d}, où d est tel que défini ;
- Z représente indépendamment une liaison ou (CH₂)ⱼ, où j est 1-4 ;
- R¹ représente indépendamment une liaison, H, alkyle en C₁-C₈, alkyle en C₁-C₈ substitué, aryle en C₅-C₁₄, aralkyle ou M^{e+} ;
- M représente indépendamment Na, K ou un ion métallique pharmaceutiquement acceptable ; et
- e = 1-3.

2. Composé selon la revendication 1, dans lequel L² représente O ; A et A¹ représentent indépendamment (CH₂)_{d}, 1,2-phénylène, 1,3-phénylène ou 1,4-phénylène ; et d représente 1-8.

3. Composé selon la revendication 1, dans lequel A et A¹ représentent (CH₂)₂ et D² est le même que D¹.

4. Composé selon la revendication 1, dans lequel D² est le même que D¹.

5. Composé selon la revendication 3, dans lequel le composé est choisi parmi : 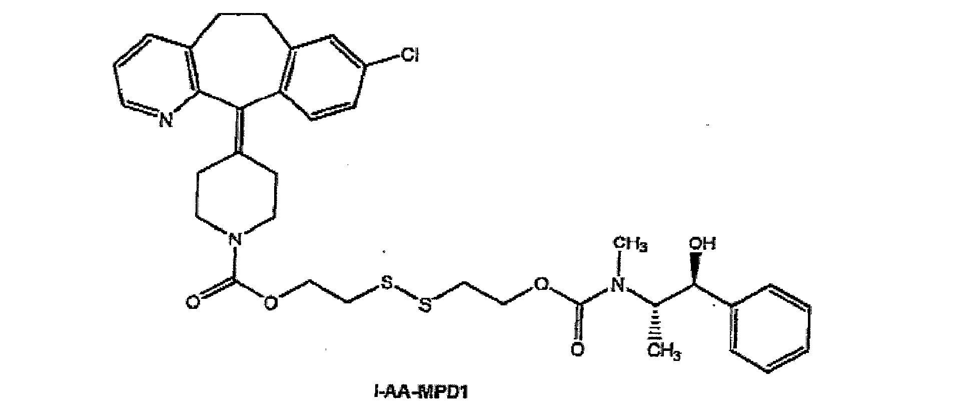 et

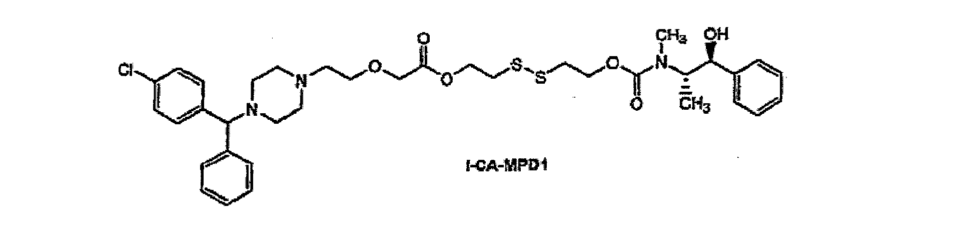

6. Composé selon la revendication 1, dans lequel le composé est choisi parmi I-CA-MPD2, I-CA-MPD3, I-CA-MPD4 et I-AH-MPD16 ou les sels pharmaceutiquement acceptables de ceux-ci.

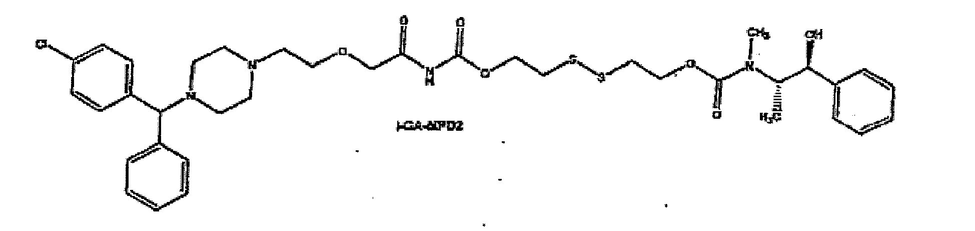

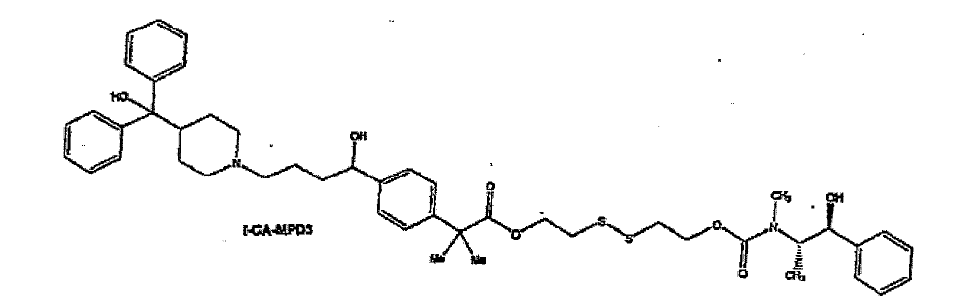

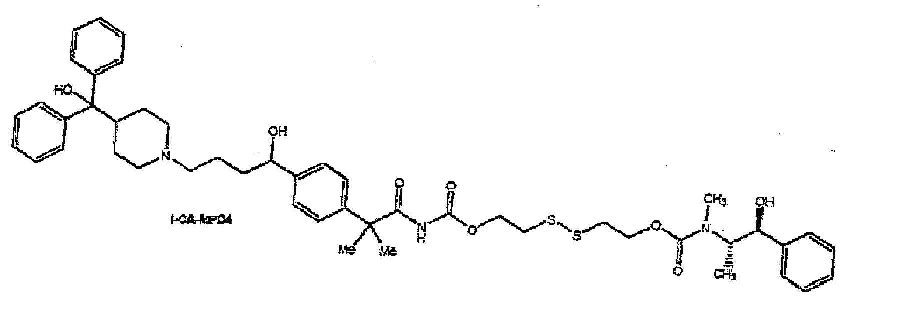

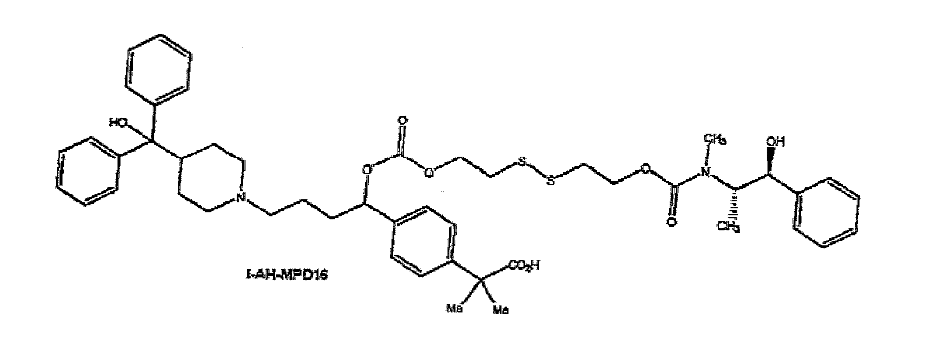

7. Composition pharmaceutique comprenant une quantité thérapeutiquement efficace du composé tel que défini à l'une quelconque des revendications 1 à 6, ou un sel pharmaceutiquement acceptable de celui-ci et un ou plusieurs supports, véhicules ou diluants pharmaceutiquement acceptables.

8. Composé selon la revendication 1, dans lequel D¹ et D² en tant que paires de médicaments sont choisis parmi : la Pseudoéphédrine et la Fexofénadine/Cétirizine/Desloratidine.

9. Composé selon l'une quelconque des revendications 1 à 6 et 8, destinée à être utilisée dans le traitement de maladies où une libération chronique, entretenue et sélective du médicament constitutif est utile.

10. Composition selon la revendication 7, destinée à être utilisée dans le traitement de maladies où une libération chronique, entretenue et sélective du ou des médicaments constitutifs est utile.

11. Intermédiaires obtenus dans la préparation de composés de la revendication 1, les intermédiaires étant choisis parmi : 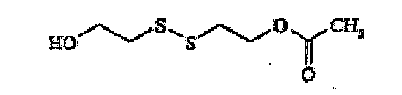 Acétate de 2-((2-hydroxyéthyl)disulfanyl)éthyle (L1-1a) 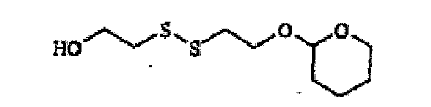 2-((2-(Tétrahydro-2H-pyran-2-yloxy)éthyl)disulfanyl)éthanol (LI-1b) 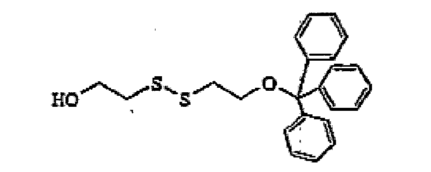 2-((2-Trityloxy)éthyl)disulfanyl)éthanol (LI-1c) 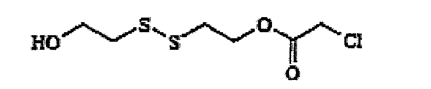 2-Chloroacétate de 2-((2-hydroxyéthyl)disulfanyl)éthyle (L1-1d) 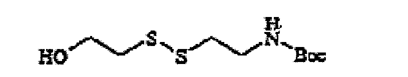 2-((2-Hydroxyéthyl)disulfanyl)éthylcarbamate de *tert*-butyle (LI-2c) 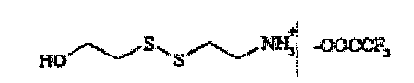 Sel de TFA de 2-((2-hydroxyéthyl)disulfanyl)-éthylamine (LI-2c.TFA) 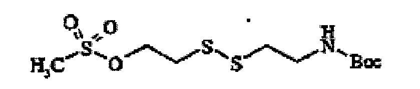 Méthanesulfonate de 2-((2-*tert*-butoxycarbonylamino)éthyl)-disulfanyl)éthyle (LI-2d) 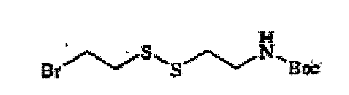 2-((2-Bromoéthyl)-disulfanyl)éthylcarbamate de *tert*-butyle (LI-2e) 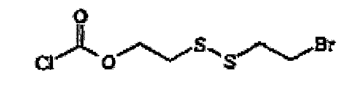 Carbonochloridate de 2-((2-bromoéthyl)disulfanyl)éthyle (LI-6) 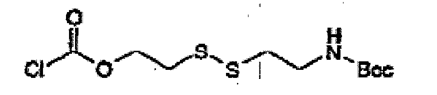 Carbonochloridate de 2-((2-tert-butoxycarbonylamino)éthyl)-disulfanyl)éthyle (LI-7) 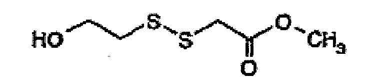 2-((2-Hydroxyéthyl)-disulfanyl)acétate de méthyle (L3I2a) 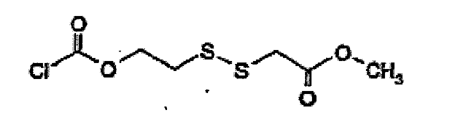 2-((2-(Chlorocarbonyloxy)-éthyl)disulfanyl)acétate de méthyle (L3I3a) 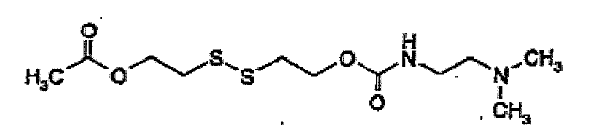 Acétate de 2-méthyl-6-oxo-7-oxa-10,11-dithia-2,5-diazatridecan-13-yle (LI-8) 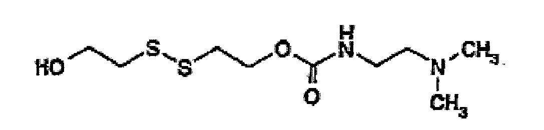 2-(Diméthylamino)éthylcarbamate de 2-((2-hydroxyéthyl)disulfanyl)éthyle (LI-9) 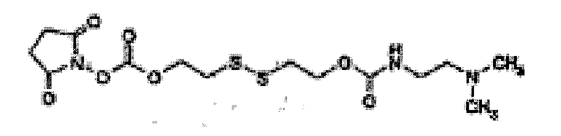 2-(Diméthylamino)éthylcarbamate de 2-((2-((2,5-dioxopyrrolidin-1-yloxy)carbonyloxy)éthyl)-disulfanyl)-éthyle (LI-10)

12. Procédé de préparation de composés tels que définis à la revendication 1, dans lequel la préparation desdits composés met enjeu l'utilisation des intermédiaires choisis dans un groupe consistant en : 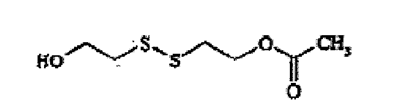 Acétate de 2-((2-hydroxyéthyl)disulfanyl)éthyle (L1-1a) 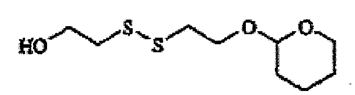 2-((2-(Tétrahydro-2*H*-pyran-2-yloxy)éthyl)disulfanyl)éthanol (LI-1b) 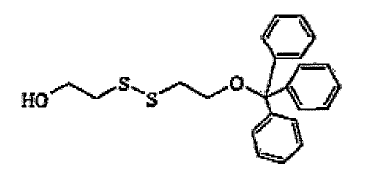 2-((2-Trityloxy)éthyl)disulfanyl)éthanol (LI-1c) 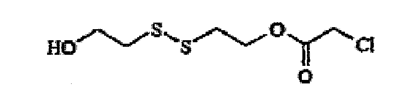 2-Chloroacétate de 2-((2-Hydroxyéthyl)disulfanyl)éthyle (LI-1d) 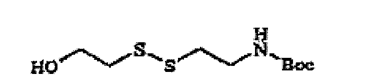 2-((2-Hydroxyéthyl)disulfanyl)éthylcarbamate de *tert*-butyle (LI-2c) 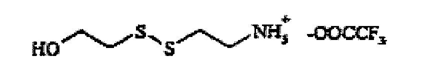 Nitrate de 2-((2-hydroxyéthyl)disulfanyl)-éthyle (LI-2c.TFA) 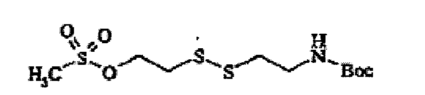 Méthanesulfonate de 2-((2-tert-butoxycarbonylamino)éthyl)-disulfanyl)éthyle (LI-2d) 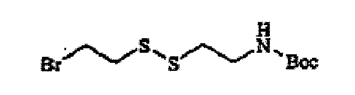 2-((2-Bromoéthyl)-disulfanyl)éthylcarbamate de *tert*-butyle (LI-2e) 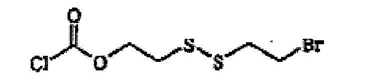 Carbonochloridate de 2-((2-bromoéthyl)disulfanyl)éthyle (LI-6) 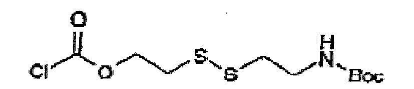 Carbonochloridate de 2-((2-tert-butoxycarbonylamino)éthyl)-disulfanyl)éthyle (L1-7) 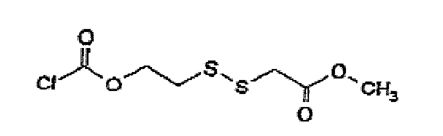 2-((2-(Chlorocarbonyloxy)-éthyl)disulfanyl)acétate de méthyle (L3I3a) 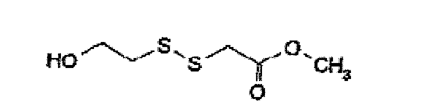 2-((2-Hydroxyéthyl)-disulfanyl)acétate de méthyle (L3I2a) 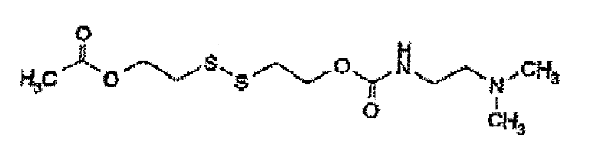 Acétate de 2-méthyl-6-oxo-7-oxa-10,11-dithia-2,5-diazatridécan-13-yle (LI-8) 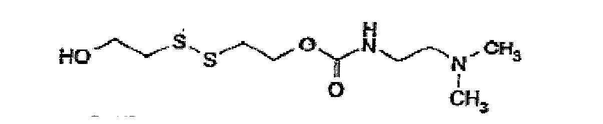 2-(Diméthylamino)éthylcarbamate de 2-((2-hydroxyéthyl)disulfanyl)éthyle (LI-9) 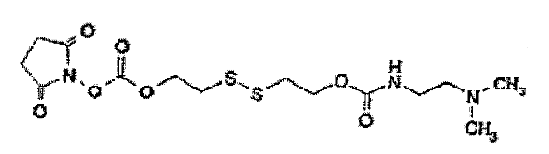 2-(Diméthylamino)éthylcarbamate de 2-((2-((2,5-dioxopyrrolidin-1-yloxy)carbonyloxy)éthyl)-disulfanyl)-éthyle (LI-10)

13. Procédé de préparation du composé tel que défini à la revendication 1, ou de sels pharmaceutiquement acceptables de celui-ci, le procédé étant choisi parmi :
Procédé 1 :
A) monoprotection du disulfure de Bis-(2-hydroxyéthyle) (SL-1) par un groupe protecteur d'hydroxyle approprié pour donner l'intermédiaire monoprotégé correspondant L1-1x 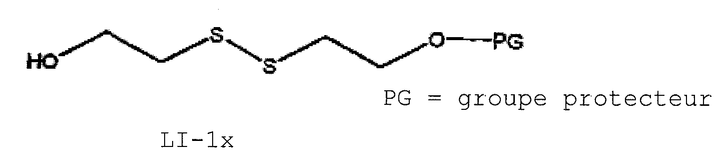
B) conversion de l'intermédiaire monoprotégé LI-1x obtenu à l'étape A en un intermédiaire formyle activé LI-1xy par traitement par du phosgène ou son équivalent ;
et
C) réaction de l'intermédiaire formyle activé LI-1xy obtenu à l'étape B avec un médicament à teneur en amino ou hydroxyle approprié (D¹) pour donner le composé correspondant de formule 1 ;
procédé 2 :
A) conversion par des méthodes connues du médicament à teneur en carboxyle (D¹) en son halogénure d'acyle ou imidazoline ou isocyanate activé ; et
B) réaction de l'intermédiaire obtenu à l'étape A) avec l'intermédiaire de liaison monoprotégé LI-1x pour obtenir le composé de formule I ;
procédé 3 :
A) monoprotection du disulfure de bis-(2-hydroxyéthyle) (SL-1) par un groupe protecteur d'hydroxyle approprié pour donner l'intermédiaire monoprotégé correspondant L1-1x ;
B) réaction de l'intermédiaire de liaison formyle LI-1xy avec un médicament à teneur en amino ou hydroxyle (D¹) pour obtenir le promédicament de formule I avec un groupe hydroxyle libre sur l'agent de liaison ;
C) conversion de l'intermédiaire obtenu à l'étape B) en dérivé halogénure de formyle activé ou imidazolide activé ; et
D) réaction de l'intermédiaire obtenu à l'étape C) avec le médicament D² pour obtenir le promédicament mutuel de formule I.
